# EUROPEAN PATENT APPLICATION

(11) **EP 3 892 281 A2**
(43) Date of publication of application: **13.10.2021**
(21) Application number: 21165556.8
(22) Date of filing: 27.10.2016
(51) Int. Cl.: A61K 31/585, A61K 31/155, A61K 31/4439, A61P 15/08

(54) **TREATMENT OF HEPATIC AND/OR VISCERAL FAT EXCESS**

(30) Priority: 27.10.2015 GB 201518979
(62) Divisional of application: 16788501.1
(71) Applicant: Katholieke Universiteit Leuven KU Leuven Research & Development, 3000 Leuven (BE); Hospital Sant Joan de Deu, 08950 Esplugues de Llobregat (ES)
(72) Inventor: IBAÑEZ, Lourdes, 08022 Barcelona (ES); DE ZEGHER, Francis, 3080 Tervuren (BE)
(74) Representative: Hoffmann Eitle

(57) **Abstract**

The present invention relates to a method and composition for use in treating a condition that benefits from the reduction of hepatic and/or visceral fat, such as polycystic ovary syndrome in adolescent girls or women of childbearing age, involving the use of spironolactone, pioglitazone and metformin.

## Description

### FIELD OF THE INVENTION

The present invention relates to a method and composition for use in treating a condition that benefits from the reduction of hepatic and/or visceral fat. The present invention further relates to a method and composition for use in the treatment or prevention of PCOS and PCOS-like conditions, including the treatment or prevention of subfertility due to a low ovulation rate associated with liver steatosis in adolescent girls or women of childbearing age.

### BACKGROUND OF THE INVENTION

Hyperinsulinaemic androgen excess, a symptom that leads to the diagnosis of polycystic ovary syndrome (PCOS), is the most common cause of hirsutism, acne, seborrhoea and menstrual irregularity in adolescent girls.¹ The term 'hyperinsulinaemic androgen excess' can be used in place of the term 'polycystic ovary syndrome', which has been criticised by an NIH panel for focusing on a criterion for diagnosing the syndrome that is neither necessary nor sufficient.
The ovarian androgen excess seems to originate most often from an absolute or a relative fat excess in adipose tissue, in particular in the liver, and from ensuing elevations in insulinaemia and gonadotrophin secretion.^{2,3} Moreover oligo-ovulatory androgen excess, which is major cause of subfertility in women, also relates to hepatic steatosis, independently of obesity.
There is no approved therapy for hyperinsulinaemic androgen excess in adolescent girls. Prime recommendation is to reduce body adiposity with lifestyle measures.^{4,5} Adding an oral oestro-progestogen contraceptive (OC) is a broadly endorsed approach.^{5,6} An alternative approach is to add an insulin-sensitising medication for those hyperandrogenic girls who are not sexually active.^{7,8} Studies hitherto comparing these two approaches in girls with androgen excess have disclosed that insulin sensitisation confers more normalising effects, including on markers of visceral adiposity, low-grade inflammation and cardiovascular health, as well as on leukocyte telomere length, a marker of cellular aging.⁹⁻¹² However, these data are of limited relevance because the tested medications (such as flutamide and cyproterone acetate) are not available in many countries.^{5,9,10}

### SUMMARY OF THE INVENTION

The present invention reports a first randomised study comparing the effects of a widely prescribed OC to those of a novel insulin sensitizing treatment involving the use of a low-dose combination of spironolactone-pioglitazone-metformin (SPIOMET) in adolescent girls with hyperinsulinaemic androgen excess with or without oral contraception. It was surprisingly observed that the SPIOMET treatment of the present invention decreased the visceral and liver fat content to a greater extent than reported in previous studies investigating the effects of alternative insulin sensitizing treatments. Moreover, no reversal of the positive effects on visceral and liver fat of the SPIOMET treatment of the present invention was observed one year post-treatment. This finding is of particular interest given the key role of visceral and in particular hepatic fat in the phenotype of PCOS. The treatment of the present invention consistently had a remarkably positive effect on the ovulation rate of adolescent girls or young women suffering from hepatic steatosis related androgen excess (PCOS) and this positive effect also persisted after termination of the treatment. As such it is a main object of the present invention to contribute to the treatment of PCOS and PCOS-like conditions, including the treatment or prevention of subfertility due to a low ovulation rate associated with liver steatosis in adolescent girls or women of childbearing age.

A first aspect of the present invention is a pharmaceutical composition comprising spironolactone, pioglitazone and metformin for use in the treatment of PCOS and PCOS-like conditions, including the treatment or prevention of subfertility due to a low ovulation rate associated with liver steatosis in adolescent girls or women of childbearing age.
Another aspect of the present invention is a method of treating PCOS and PCOS-like conditions, including the treatment or prevention of subfertility due to a low ovulation rate associated with liver steatosis in adolescent girls or women of childbearing age, said method comprising the administration of a combination of spironolactone, pioglitazone and metformin.
A further aspect of the present invention is a pharmaceutical composition comprising spironolactone, pioglitazone and metformin for use in treating a condition that benefits from the reduction of hepatic and/or visceral fat. In particular embodiments, such reduction of hepatic and/or visceral fat occurs in absence of a concomitant reduction of total body weight, of body weight, lean mass and/or total fat mass.
A related aspect of the present invention is method for treating a condition that benefits from a reduction of hepatic and/or visceral fat, said method comprising the combined administration of spironolactone, pioglitazone and metformin. In particular embodiments, such reduction of hepatic and/or visceral fat occurs in absence of a concomitant reduction of total body weight, of body weight, lean mass and/or total fat mass.
An even further aspect of the present invention is a pharmaceutical delivery form, preferably an oral delivery form, comprising spironolactone, pioglitazone and metformin in each individual unit of said delivery form. More preferably an individual unit of the delivery form, preferably an oral delivery form, comprising the required daily dose of each of spironolactone, pioglitazone and metformin.

### DETAILLED DESCRIPTION

### Description of the Figures

**Figure 1****:** Numbers of inferred ovulations over 12+12 post-treatment weeks associated with on-treatment changes of hepatic fat, and to post- versus pre-treatment changes of free testosteronaemia. Results from OC girls (N=17) are shown in black circles, and those from SPIOMET girls in white circles (N=17). P and R values are from Pearson correlations.
**Figure 2****:** Representative post-treatment patterns of menstrual history, salivary progesterone, and inferred ovulations in adolescent girls with PCOS, who received OC or SPIOMET treatment for 12 months. Data were collected in two time windows of 12 weeks (plus two more weeks of menstrual history) between 3-6 months post-treatment, and again between 9-12 months post-treatment. Girls with similar menstrual histories may have markedly different ovulation rates.

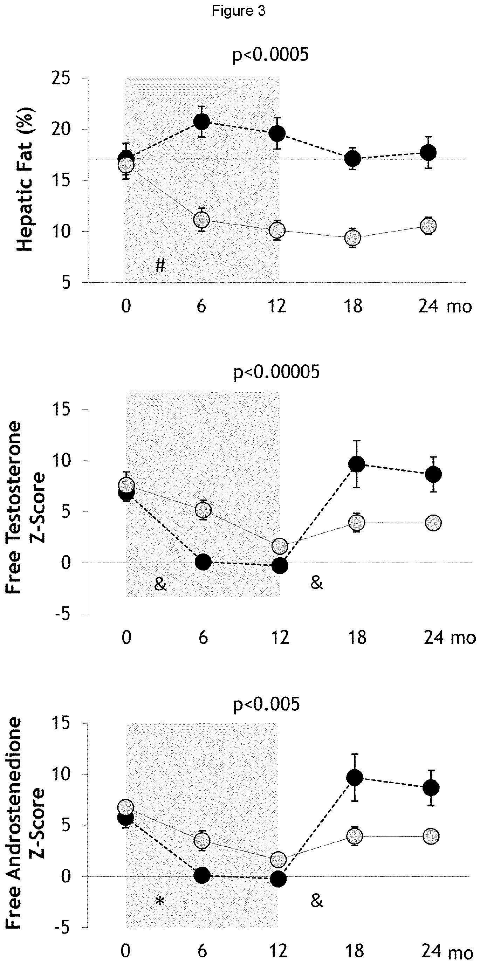

**Figure 3****:** Longitudinal changes in hepatic fat and free androgenaemia Z-scores in adolescent girls with PCOS, who received an OC (black circles; N=17) or low-dose SPIOMET (white circles; N=17) for 12 months, and who remained subsequently untreated for 12 months. OC and SPIOMET had opposing effects on hepatic fat; OC lowered free androgenaemia faster than SPIOMET, but was also followed by a faster rebound. Results are expressed as means and SEM. P values refer to differences between subgroups (0-24 months). Symbols along the X-axis (*p<0.05, &p<0.01, #p<0.001) refer to differential changes between 0-6 months and between 12-18 months.
**Figure 4****:** Scheme presenting the treatment method of the present invention for preventing or treating subfertility due to low ovulation rates associated with liver steatosis in adolescent girls.
**Figure 5****:** Scheme presenting the treatment method of the present invention for preventing or treating subfertility due to low ovulation rates associated with liver steatosis in women of childbearing age.
**Figure 6****:** Flow diagram presenting the course of the study of Example 1.
**Figure 7****:** Representation of extremes of post-treatment ovulatory patterns in adolescent girls with polycystic ovary syndrome.

### Description

Within the context of the present invention spironolactone refers to a synthetic steroidal compound with a structure as indicated below as well as derivatives thereof. In most countries spironolactone is marketed within a drug formulation branded as Aldactone®.

### Structural formula of spironolactone:

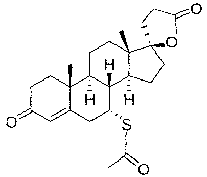

Systematic name spironolactone: 7a-acetylthio-3-oxo-17a-pregn-4-ene-21,17-carbolactone or 17-hydroxy-7a-mercapto-3-oxo-17a-pregn-4-ene-21-carboxylic acid, γ-lactone acetate

Within the context of the present invention pioglitazone refers to a compound of the class thiazolidinedione with a structure as indicated below as well as derivatives thereof. In most countries pioglitazone is marketed within a drug formulation branded as Actos®.

### Structural formula of pioglitazone:

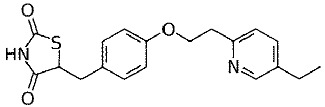

Systematic name pioglitazone: (RS)-5-(4-[2-(5-ethylpyridin-2-yl)ethoxy]benzyl)thiazolidine-2,4-dione.

Within the context of the present invention metformin refers to a compound with a structure as indicated below as well as derivatives thereof. In most countries metformin is marketed within a drug formulation branded as Metformina® or Glucophage®.

### Structural formula of metformin:

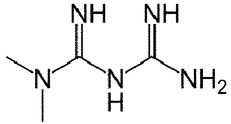

Systematic name metformin: N,N-Dimethylimidodicarbonimidic diamid

Within the context of the present invention ovulation rate refers to the occurrence of spontaneous ovulations over a given period of time, for instance three months. A low ovulation rate refers to a situation wherein the ovulations per month are below 0.7, for instance below 0.6 such as below 0.5 or below 0.4.
Within the context of the present invention liver steatosis refers to a condition associated with the accumulation of fat in the liver. Within the particular context of the present invention liver steatosis is not related to alcohol consumption, this type of liver steatosis is generally referred to as Non-alcoholic fatty liver disease (NAFLD).
In humans, a woman's fertility peaks in the early and mid-20s, after which it starts to decline slowly, with a more dramatic drop at around 35. Menopause, or the cessation of menstrual periods, generally occurs in the 40s and 50s and marks the cessation of fertility. Therefore, within the context of the present invention childbearing age preferably refers to an age between 20 and 45 years old, more preferably to an age between 20 and 35 years old.

Oligo-ovulatory androgen excess in adolescent girls or women (a further term that can be used to refer to polycystic ovary syndrome [PCOS] according to NIH) is a major cause of subfertility and relates to hepatic steatosis, independently of obesity. PCOS is further associated with the occurrence of following symptoms: hirsutism, acne, seborrhoea and menstrual irregularity. It is unknown whether early interventions influence the post-treatment rates of spontaneous ovulations. However, in the context of the present invention the inventors observed a dramatic increase of the post-treatment ovulation rates after randomized interventions for hepatic steatosis associated androgen excess in (mostly non-obese) adolescent girls/young women within the group treated with a combination of spironolactone, pioglitazone and metformin as compared to the group treated with a standard treatment consisting of the administration of an oral contraceptive

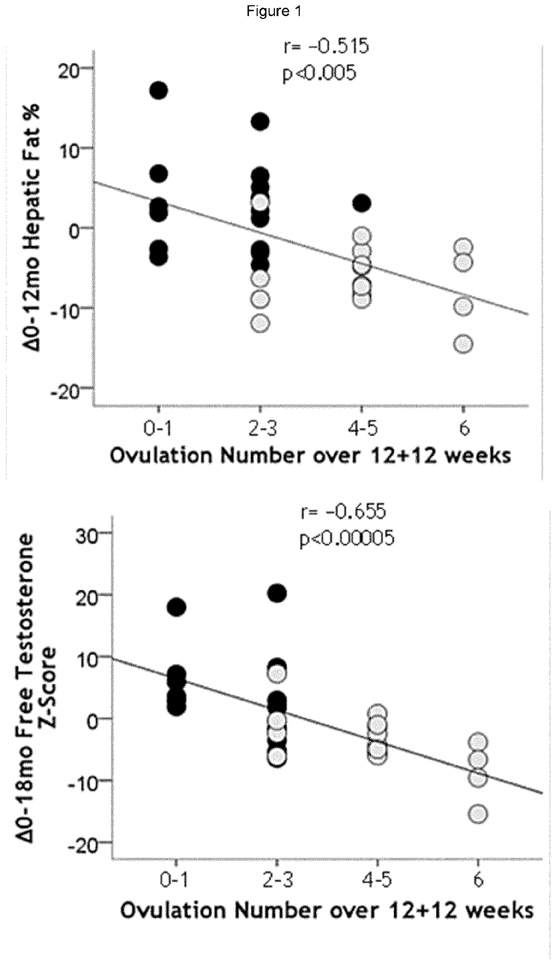

The present invention reports a first randomised study comparing the effects of a widely prescribed OC to those of an insulin sensitizing treatment involving the use of a low-dose combination of spironolactone-pioglitazone-metformin (SPIOMET) in adolescent girls with hyperinsulinaemic androgen excess, also referred to as PCOS. It was surprisingly observed that the SPIOMET treatment of the present invention decreased the visceral and liver fat content to a greater extent than reported in previous studies investigating the effects of alternative insulin sensitizing treatments, such as the combined administration of pioglitazone, flutamide and metformin¹⁷. In addition, the SPIOMET treatment of the present invention gradually normalized the occurrence of endogenous regular menses as well as the markers of androgenaemia and insulinaemia (Table 3). Moreover, no reversal of the positive effects of the SPIOMET treatment on insulinaemia markers, visceral and liver fat was observed one year post-treatment, while the post-treatment return of hyperandrogenaemia was markedly slower than observed after the termination of the standard OC treatment. A further surprising and important finding of the present invention was that the post-treatment normalization of ovulation rates significantly correlated with the reduction of liver fat (fig. 1). Accordingly, the reduction of liver fat following the SPIOMET treatment was associated with a normalisation of ovulation rates during a one year post-treatment period. This finding is very important in view of preventing or treating the sub- or infertility associated with PCOS notwithstanding that conception has to be avoided during spironolactone or pioglitazone intake. Indeed, the long-term post-treatment normalisation of ovulation rates provides adequate time for PCOS patients to attempt conceiving in a safe, and preferably natural, manner after the termination of the SPIOMET intake. Furthermore, the observation that excessive liver fat can cause a reduction in ovulation rates provides treatment options for women with liver steatosis, not linked to PCOS, who suffer from infertility, such as for instance in women that are overweight and/or have excessive ectopic fat deposition. Indeed the SPIOMET treatment according to the present invention is also advised as part of a treatment for restoring fertility in this patient group, preferably in combination with the adoption of life-style changes promoting weight loss and favouring increased physical activity.

A first aspect of the present invention is a pharmaceutical composition comprising spironolactone, pioglitazone and metformin for use in the prevention or treatment of PCOS or oligo-ovulatory androgen excess in adolescent girls or women of childbearing age. Typically, the pharmaceutical composition of the present invention is used in the treatment of adolescent girls or women of childbearing age who also exhibit androgen excess and/or hyperinsulinemia, low adiponectin, high C-reactive protein levels and/or high gonadotropin levels. Generally, these girls and women of childbearing age also exhibit liver and visceral fat excess and/or dyslipidemia. The occurrence of these PCOS symptoms may or may not be associated with overweight or obesity. Typically, the use of said pharmaceutical composition according to the first aspect of the present invention alleviates a single, several or all of said PCOS associated symptoms. For instance, said use may alleviate or attenuate any one, several or all of the following PCOS symptoms: hyperinsulinemia, androgen excess, excessive liver fat, excessive visceral fat, irregular endogenous menses and low ovulation rate. Furthermore, this alleviation or attenuation of any one, several or all of said symptoms was observed to persist after the termination of the use of said pharmaceutical composition. In particular the reduction of excessive liver fat and visceral fat and the normalisation of ovulation rate were maintained for a prolonged period post-treatment, such as during a period of 6 or 12 months following the termination of the use of said pharmaceutical composition.
A second aspect of the present invention is a pharmaceutical composition combining spironolactone, pioglitazone and metformin for use in the prevention or treatment of low ovulation rate related to liver steatosis in adolescent girls or women of childbearing age. Within the population under treatment this liver steatosis is not necessarily associated with overweight or obesity. Typically, the pharmaceutical composition of the present invention is used in the treatment of adolescent girls or women of childbearing age who also exhibit androgen excess and hyperinsulinemia, low adiponectin, high C-reactive protein levels and/or high gonadotropin levels. Generally, these girls and women of childbearing age also exhibit liver and visceral fat excess and/or dyslipidemia

A particular embodiment of the second aspect of the present invention is a pharmaceutical composition combining spironolactone, pioglitazone and metformin for use in the prevention or treatment of low ovulation rate in adolescent girls or women of childbearing age suffering from oligo-ovulatory androgen excess or PCOS. The positive effects of the use of this pharmaceutical composition on ovulation rate clearly correlated with a preceding reduction of liver fat. In consequence, this use of said pharmaceutical composition is particularly suited for the treatment of low ovulation rates in PCOS patients having excess liver fat or liver steatosis.
A further embodiment of the second aspect of the present invention is a pharmaceutical composition combining spironolactone, pioglitazone and metformin for use in the prevention or treatment of low ovulation rate associated with liver steatosis in adolescent girls or women of childbearing age that are not considered to suffer from oligo-ovulatory androgen excess or PCOS. Typically the adolescent girls or women of childbearing age within this patient group are overweight and/or exhibit excessive ectopic fat deposition, in particular excessive visceral and liver fat deposition.

A third aspect of the present invention is a pharmaceutical composition comprising spironolactone, pioglitazone and metformin for use in treating a condition that benefits from the reduction of hepatic and/or visceral fat. In particular embodiments, such reduction of hepatic and/or visceral fat occurs in absence of a concomitant reduction of total body weight, of body weight, lean mass and/or total fat mass. The condition may be polycystic ovary syndrome, metabolically obese normal weight syndrome (De Lorenzo syndrome), metabolic syndrome, obesity or being overweight. Preferably, this treatment is combined with the adoption of life-style involving a balanced diet and favouring increased physical activity.

A particular embodiment of the third aspect of the present invention is a pharmaceutical composition comprising spironolactone, pioglitazone and metformin for use in treating a condition that benefits from the reduction of hepatic and/or visceral fat in adolescent girls or women of childbearing age. In particular embodiments, such reduction of hepatic and/or visceral fat occurs in absence of a concomitant reduction of total body weight, of body weight, lean mass and/or total fat mass. The condition may be polycystic ovary syndrome, metabolically obese normal weight syndrome (De Lorenzo syndrome), metabolic syndrome, obesity or being overweight. Preferably, this treatment is combined with the adoption of life-style involving a balanced diet and favouring increased physical activity.

The term "overweight" has been defined by the U.S. National Institutes of Health (NIH) and the World Health Organization (WHO) as a BMI of 25 or more for whites, Hispanics and blacks. For Asians, "overweight" is defined as a BMI of between 23 and 29.9. For children and adolescents, age-, sex-, ethnicity- and/or country-specific references must be taken into account, so that "overweight" for children and adolescents generally corresponds to a BMI above the 85th percentile.
"Obesity" has been defined as a BMI of 30 or more for all groups. However, for children and adolescents "obesity" is defined as a BMI greater than or equal to the 95th percentile.
According to the U.S. NIH, "metabolic syndrome" is the name for a group of risk factors that increases your risk of heart disease and other health problems, such as diabetes and stroke. The term "metabolic" refers to the biochemical processes involved in the body's normal functioning. Risk factors are traits, conditions, or habits that increase your chance of developing a disease. You must have at least three metabolic risk factors to be diagnosed with metabolic syndrome. These include: a large waistline or abdominal obesity; a high triglyceride level; a low HDL cholesterol level; high blood pressure; and high fasting blood sugar.

It is preferred that said pharmaceutical composition for use in accordance with the present invention is combined with the use of an oral or non-oral contraceptive therapy by said adolescent girls or women of childbearing age. In particular embodiments, the contraceptive is a non-oral contraceptive, such as an intra-uterine contraceptive, for instance an intra-uterine coil.

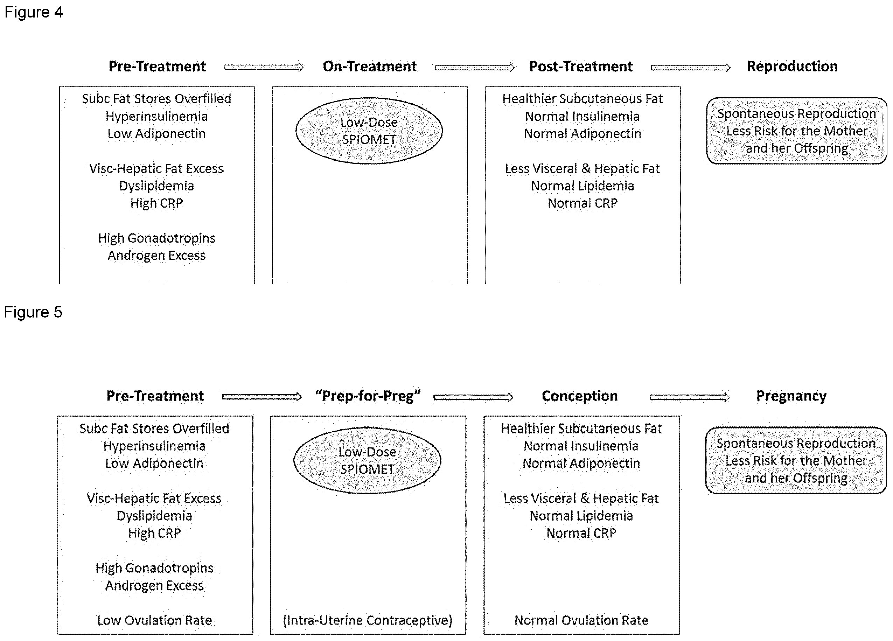

The pharmaceutical composition for use in accordance with the present invention typically comprises at least 25, preferably at least 30, such as at least 40 or at least 45 mg spironolactone in a daily dose of said composition. In addition the pharmaceutical composition for use in accordance to with the present invention typically comprises at most 100, preferably at most 75, such as at most 70 or 60 mg spironolactone in a daily dose of said composition. In a particular embodiment the pharmaceutical composition for use in accordance with the present invention comprises 50 mg spironolactone in a daily dose of said composition.
The pharmaceutical composition for use in accordance with the present invention typically comprises at least 2.5, preferably at least 5, such as at least 6 or at least 7 mg pioglitazone in a daily dose of said composition. In addition the pharmaceutical composition for use in accordance with the present invention typically comprises at most 15, preferably at most 10, such as at most 9 or 8 mg pioglitazone in a daily dose of said composition. In a particular embodiment the pharmaceutical composition for use in accordance to with the present invention comprises 7.5 mg pioglitazone in a daily dose of said composition.
The pharmaceutical composition for use in accordance with the present invention typically comprises at least 500, preferably at least 600, such as at least 700 or at least 800 mg metformin in a daily dose of said composition. In addition the pharmaceutical composition for use in accordance with the present invention typically comprises at most 1500, preferably at most 1200, such as at most 1000 or 900 mg metformin in a daily dose of said composition. In a particular embodiment the pharmaceutical composition for use in accordance with the present invention comprises 850 mg metformin in a daily dose of said composition.
It is further preferred that a daily dose of the pharmaceutical composition according to the present invention comprising the required daily dose of each spironolactone, pioglitazone and metformin is formulated in a single unit of an oral delivery form, such as a single tablet or capsule, which can be readily ingested.
As such, the use of the pharmaceutical composition according to the present invention may contribute to conception, preferably natural conception, once said adolescent girls reach adulthood and childbearing age (see Figure 4). In women of childbearing age, in particularly those suffering from oligo-ovulatory androgen excess related to hepatic steatosis, the use of the pharmaceutical composition according to the present invention results in an attenuation of oligo-ovulatory subfertility following a reduction of ectopic fat, in particular of hepatic fat (Figure 5). Preferably, the use of the pharmaceutical composition according to the present invention in adolescent girls or women of childbearing age is maintained until a sufficient reduction of the ectopic fat is observed. Preferably, such sufficient reduction of ectopic fat corresponds to a reduction of the estimated liver fat content with at least 10%, more preferably with at least 20% or 30%, such as for instance a reduction of 40% or 50% or more as compared to the estimated liver fat content at the start of the treatment. Typically such sufficient reduction of ectopic fat is associated with a reduction of the estimated visceral fat with at least 10%, more preferably of with least 15%, such as for instance a reduction of 20% or 30% or more as compared to the estimated visceral fat a the start of the treatment.

Considering that the stimulatory effects on ovulation rate of the use of the pharmaceutical composition according to present invention persist after terminating the intake of said composition it can be considered to terminate its intake, preferably once such sufficient reduction of ectopic fat is attained, when conception is desired in order to avoid any putative interference of these compounds with the embryonal or foetal development.
Preferably, the use of the pharmaceutical composition according to the present invention is combined with the use of an oral or non-oral contraceptive, in particular a non-oral contraceptive, such as an intra-uterine, contraceptive. The use of said non-oral contraceptive has the advantage that it avoids conception and pregnancy during the intake of the medicinal compounds contained in said composition and thus avoids any putative interference of these compounds on the embryonal and/or foetal development. Preferably once a sufficient reduction of ectopic fat has been achieved and conception is desired the use of said oral or non-oral contraceptive, preferably a non-oral contraceptive, can be abandoned following the termination of the combined administration of spironolactone, pioglitazone and metformin through said composition.

A fourth aspect of the present invention is a method for the prevention or treatment of oligo-ovulatory androgen excess or PCOS in adolescent girls or women, said method comprising the combined administration of spironolactone, pioglitazone and metformin. Typically, this method of the present invention is used in the treatment of adolescent girls or women of childbearing age who also exhibit hyperinsulinemia, low adiponectin, high C-reactive protein levels and/or high gonadotropin levels. Generally, these girls and women of childbearing age also exhibit liver and visceral fat excess and/or dyslipidemia. The occurrence of these PCOS symptoms may or may not be associated with overweight or obesity. Typically, the method according to this third aspect of the present invention alleviates a single, several or all of said PCOS associated symptoms. For instance, said method may alleviate or attenuate any one, several or all of the following PCOS symptoms: hyperinsulinemia, androgen excess, excessive liver fat, excessive visceral fat, irregular endogenous menses and low ovulation rate. Furthermore, this alleviation or attenuation of any one, several or all of said symptoms was observed to persist after the termination of the use of said pharmaceutical composition. In particular the reduction of excessive liver fat and visceral fat and the normalisation of ovulation rate were maintained for a prolonged period post-treatment, such as during a period of 6 or 12 months following the termination of the administration of spironolactone, pioglitazone and metformin.

A fifth aspect of the present invention provides a method for preventing or treating low ovulation rate associated with liver steatosis in adolescent girls or women of childbearing age, said method comprising the administration of a combination of spironolactone, pioglitazone and metformin.

A particular embodiment of the fifth aspect of the present invention is a method for preventing or treating low ovulation rate in adolescent girls or women of childbearing age suffering from oligo-ovulatory androgen excess or PCOS, said method comprising the combined administration of spironolactone, pioglitazone and metformin. The positive effects of this method on ovulation rate clearly correlated with a treatment associated preceding reduction of liver fat. In consequence, this method is particularly suited for the treatment of low ovulation rates in PCOS patients having excess liver fat or liver steatosis.

A further embodiment of the fifth aspect of the present invention is a method for preventing or treating low ovulation rate associated with liver steatosis in adolescent girls or women of childbearing age that are not considered to suffer from oligo-ovulatory androgen excess or PCOS said method comprising the combined administration of spironolactone, pioglitazone and metformin. Typically the adolescent girls or women of childbearing age within this patient group are overweight and/or exhibit excessive ectopic fat deposition, in particular excessive visceral and liver fat deposition.

A sixth aspect of the present invention is method for treating a condition that benefits from a reduction of hepatic and/or visceral fat, said method comprising the combined administration of spironolactone, pioglitazone and metformin. In particular embodiments, such reduction of hepatic and/or visceral fat occurs in absence of a concomitant reduction of total body weight, of body weight, lean mass and/or total fat mass. The condition may be metabolically obese normal weight syndrome (De Lorenzo syndrome), metabolic syndrome, obesity or being overweight. Preferably, this treatment is combined with the adoption of life-style involving a balanced diet and favouring increased physical activity.

A particular embodiment of the sixth aspect of the present invention is method for treating a condition that benefits from a reduction of hepatic and/or visceral fat in adolescent girls or women of childbearing age, said method comprising the combined administration of spironolactone, pioglitazone and metformin. In particular embodiments, such reduction of hepatic and/or visceral fat occurs in absence of a concomitant reduction of total body weight, of body weight, lean mass and/or total fat mass. The condition may be polycystic ovary syndrome, metabolically obese normal weight syndrome (De Lorenzo syndrome), metabolic syndrome, obesity or being overweight. Preferably, this treatment is combined with the adoption of life-style involving a balanced diet and favouring increased physical activity.

In adolescent girls, particularly those exhibiting symptoms of hyperinsulinaemic androgen excess, the treatment methods according to the present invention leads to the reduction of ectopic fat, in particular hepatic fat, and an attenuation of low ovulation rate associated subfertility in (early) adulthood. As such the treatment methods of the present invention may contribute to conception, preferably natural conception, once said adolescent girls reach adulthood and childbearing age (see Figure 4). In women of childbearing age, in particularly those suffering from oligo-ovulatory androgen excess related to hepatic steatosis, the treatment methods according to the present invention result in an attenuation of oligo-ovulatory subfertility following a reduction of ectopic fat, in particular of hepatic fat (Figure 5). Preferably, the treatment of adolescent girls and/or women of childbearing age according to the treatment methods of the present invention involves maintaining the administration of the combination of spironolactone, pioglitazone and metformin until a sufficient reduction of the ectopic fat is observed. Preferably, such sufficient reduction of ectopic fat corresponds to a reduction of the estimated liver fat content with at least 10%, more preferably with at least 20% or 30%, such as for instance a reduction of 40% or 50% or more as compared to the estimated liver fat content at the start of the treatment. Typically such sufficient reduction of ectopic fat is associated with a reduction of the estimated visceral fat with at least 10%, more preferably of with least 15%, such as for instance a reduction of 20% or 30% or more as compared to the estimated visceral fat at the start of the treatment. Considering that the stimulatory effects on ovulation rate of the treatment according to present invention persist after terminating the intake of the combination of spironolactone, pioglitazone and metformin it can be considered to terminate the intake of these compounds, preferably once such sufficient reduction of ectopic fat is attained, when conception is desired in order to avoid any putative interference of these compounds with the embryonal or foetal development.
The treatment methods of the present invention are considered to be effective in adolescent girls and/or women of childbearing age exhibiting liver steatosis irrespective whether this is associated with obesity or overweight or not.
The method of the present invention can be used in the treatment of adolescent girls or women of childbearing age who exhibit androgen excess and hyperinsulinemia, next to low adiponectin, high C-reactive protein levels and/or high gonadotropin levels. Generally, these adolescent girls and women of childbearing age also exhibit visceral fat excess and/or dyslipidemia.
The treatment methods of the present invention typically involve the daily administration of at least 25, preferably at least 30, such as at least 40 or at least 45 mg spironolactone. In addition the treatment methods of the present invention typically involve the daily administration of at most 100, preferably at most 75, such as at most 70 or 60 mg spironolactone. In a particular embodiment the treatment methods of the present invention comprise the daily administration of 50 mg spironolactone.
The treatment methods of the present invention typically involve the daily administration of at least 2.5, preferably at least 5, such as at least 6 or at least 7 mg pioglitazone. In addition the treatment methods of the present invention typically involve the daily administration of at most 15, preferably at most 10, such as at most 9 or 8 mg pioglitazone. In a particular embodiment, the treatment methods of the present invention comprise the daily administration of 7.5 mg pioglitazone in a daily dose of said composition.
The treatment methods of the present invention typically involve the daily administration of at least 500, preferably at least 600, such as at least 700 or at least 800 mg metformin in a daily dose of said composition. In addition, the treatment methods of the present invention typically involve the daily administration of at most 1500, preferably at most 1200, such as at most 1000 or 900 mg metformin in a daily dose of said composition. In a particular embodiment the treatment methods of the present invention comprise the daily administration of 850 mg metformin in a daily dose of said composition.
In a preferred embodiment, the present invention comprises the daily administration of 60 to 70 mg spironolactone, 8 to 9 mg pioglitazone, and 800 to 9000 mg metformin. Preferably the present invention comprises the daily administration of 50 mg spironolactone, 7.5 mg pioglitazone, and 850 mg metformin.
It is further preferred that the treatment methods of the present invention comprise the administration of a pharmaceutical composition being an oral delivery form comprising spironolactone, pioglitazone and metformin in each individual unit of said oral delivery form, for instance in each individual tablet or capsule. More preferably an individual unit of said oral delivery form comprises the required daily dose of each spironolactone, pioglitazone and metformin. Typically, said individual unit of the oral delivery form, such as a single tablet or capsule, can be readily ingested. The tablet or capsule may be a film-coated capsule or tablet prepared according to any method known in the art by the skilled person. Preferably the capsule or tablet is formulated as an immediate release delivery form.
In a further embodiment of the present invention, spironolactone, pioglitazone and metformin are each administered sequentially in separate delivery forms. Spironolactone, pioglitazone and metformin in separate delivery forms may form a combination.
In a still further embodiment, two of spironolactone, pioglitazone and metformin are administered in a single delivery form and the remaining compound is administered in a separate delivery form.
The single delivery forms may be administered at the same time or within 5 minutes to 1 hour of each other, preferably within 15 minutes to 30 minutes of each other. Preferably, the treatment method of the present invention, the combined administration of spironolactone, pioglitazone and metformin, is combined with the use of a contraceptive therapy, in particular a non-oral conctraceptive such as an intra-uterine contraceptive.
In a further embodiment of the present invention, the combined administration of spironolactone, pioglitazone and metformin is combined with an oral contraceptive in a single delivery form.
In a further embodiment, two or three of spironolactone, pioglitazone, metformin and the oral contraceptive are administered in a single delivery form and the remaining compound or compounds are administered in a separate delivery form or forms.
The use of contraceptive therapy has the advantage that it avoids pregnancy during the intake of said medicinal compounds and thus avoids any putative interference of these compounds on the embryonal and/or foetal development. Preferably once a sufficient reduction of ectopic fat has been achieved and conception is desired the use of said non-oral contraceptive can be abandonned following the termination of the combined administration of spironolactone, pioglitazone and metformin.
A further aspect of the present invention is a pharmaceutical composition wherein the pharmaceutical composition comprises between 25 and 100 mg spironolactone, and/or between 5 and 15 mg pioglitazone and/or between 500 and 1500 mg metformin. In one embodiment, the pharmaceutical composition comprises between 25 and 100 mg spironolactone and between 5 and 15 mg pioglitazone.
In a further embodiment, the daily dose of said pharmaceutical composition comprises between 25 and 100 mg spironolactone, between 5 and 15 mg pioglitazone, and between 500 and 1500 mg metformin. In a particularly preferred embodiment, the daily dose of the pharmaceutical composition comprises 50 mg spironolactone, 7.5 mg pioglitazone, and 850 mg metformin.
In one embodiment, the pharmaceutical composition is in an oral delivery form and an individual unit of the oral delivery form comprises between 25 and 100 mg spironolactone, between 5 and 15 mg pioglitazone and between 500 and 1500 mg metformin. In a preferred embodiment, the individual unit of the oral delivery form is a tablet or capsule.

### Example 1: Low Dose Spironolactone Pioglitazone Metformin treatment for Adolescent girls/young women with hyperinsulinaemic androgen excess.

### Methods

### Study Design and Population

Table 1 summarizes the design of this randomised, single-center, open-label study over 24 months.

The study population consisted of 36 Catalan girls meeting the four inclusion criteria of hirsutism (score >8 on Ferriman-Gallwey scale), oligomenorrhea (menstrual intervals >45 d), gynaecological age (or timespan post-menarche) >2.0 yr, and absence of sexual activity (no need for contraception).

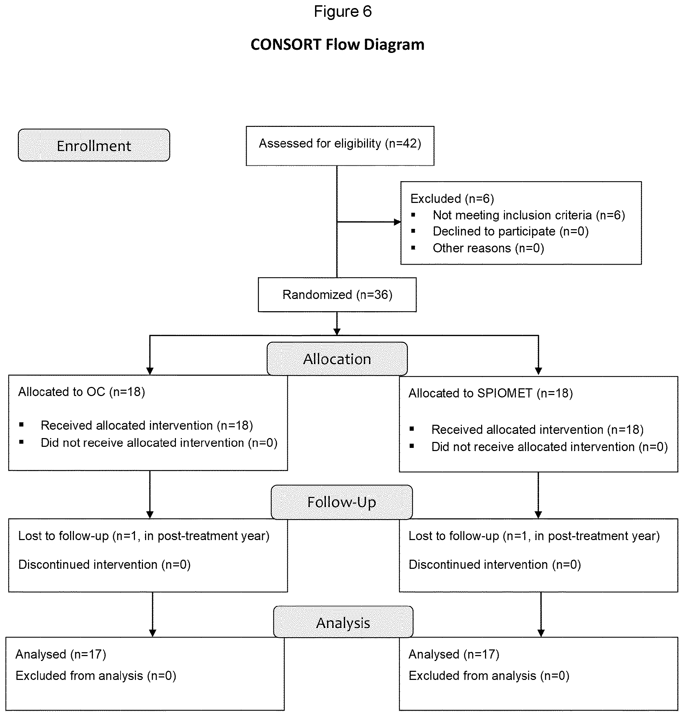

The girls were recruited in the Adolescent Endocrinology Unit of Sant Joan de Déu University Hospital, Barcelona, Spain, between January 2013 and May 2014 (CONSORT Flow Diagram; Figure 6). Recruitment was biased against overweight/obesity because, in our setting, overweight/obese adolescent girls are primarily referred to the Obesity Unit rather than to the Adolescent Endocrinology Unit.

All girls meeting the four inclusion criteria were enrolled since none of them was excluded by one of the following criteria: 21-hydroxylase deficiency (as judged by 17-OH-progesteronaemia ≥200 ng/dL in the follicular phase or after 2 months of amenorrhea); glucose intolerance or diabetes mellitus; evidence of thyroid, liver or kidney dysfunction; hyperprolactinaemia; any prior use of medication affecting gonadal or adrenal function, or carbohydrate or lipid metabolism.¹⁷

### Endpoints

The primary endpoint was post-treatment ovulation rate. Secondary outcomes were hirsutism and acne scores, body composition, abdominal fat partitioning (subcutaneous, visceral, and hepatic fat), carotid intima-media thickness (cIMT), and circulating insulin, testosterone, androstenedione, C-reactive protein (CRP), lipids, and high-molecular-weight (HMW) adiponectin.

### Study Registration and Ethics

The study was registered as ISRCTN29234515 and conducted after approval by the Institutional Review Board of Sant Joan de Déu University Hospital, after written consent by parents, and after assent by each of the study girls, including of the healthy controls who allowed to derive indicative values. Updates of the original study registration reflect changes that occurred since August 2012, notably in the diagnosis of adolescent PCOS,⁶ in the study duration (extension of the post-treatment timespan from 6 to 12 months, after obtaining an extension of funding), and in the measurement of circulating androgens (switch to liquid chromatography - tandem mass spectrometry).

### Randomisation and Study Medications

Mediterranean diet and regular exercise were recommended to all girls. Randomisation (1:1) for study medication was web-based (http://www.SealedEnvelope.com), using random permuted blocks, with strata for age (<16.0 or ≥16.0 years) and body mass index (BMI,<24.0 or ≥24.0 Kg/m²). Girls were randomly assigned to receive once daily, at dinner time, either Loette Diario (Pfizer, Madrid, Spain; 20 mcg ethinyloestradiol plus 100 mg levonorgestrel for 21/28 days, and placebo for 7/28 days) or SPIOMET, a low-dose combination of three separate generics: 50 mg spironolactone (half of 100 mg tablet of Aldactone from Pfizer, Madrid, Spain), 7.5 mg pioglitazone (half of 15 mg tablet of Actos from Takeda, Madrid, Spain), and 850 mg metformin (850 mg tablet of Metformina from Sandoz, Barcelona, Spain).

Adherence to treatment was screened by history at each clinical assessment, and by pill counts in the pharmacy delivering the study medications.

### Clinical & Endocrine-Metabolic Assessments

One investigator (LI, unblinded to treatment) measured weight, height (Harpenden stadiometer) and waist circumference, scored the presence of hirsutism (Ferriman-Gallwey) and acne (Leeds grading scale), and screened for side effects. Systolic and diastolic blood pressure were recorded on the right arm after a 5-minute rest with the girl supine, using an electronic sphygmomanometer (767 series, Welch Allyn, Spain).

Endocrine-metabolic assessments were performed in the early morning, between cycle days 3-7 (all girls but one) or after 2 months of amenorrhea (one girl). Fasting glycaemia and circulating insulin (together allowing for calculation of Homeostasis Model Assessment of Insulin Resistance, HOMA-IR), sex hormone-binding globulin (SHBG), testosterone, androstenedione, LDL- and HDL-cholesterol, triglycerides, HMW adiponectin and CRP were assessed along with safety markers alanine transaminase (ALT), aspartate transaminase (AST), and gamma glutamyl transferase (GGT). Additional safety markers were blood count and circulating urea and creatinine (data not shown). Oral glucose tolerance tests (oGTT) with 75 g glucose were performed after 3 days on a high carbohydrate diet (300 g/day) and an overnight fast, with blood sampling before glucose intake, and 30, 60, 90 and 120 min thereafter, for glucose and insulin measurements from which mean glycaemia and insulinaemia Z-scores were derived.¹⁷

### Carotid Intima-Media Thickness (cIMT)

Longitudinal ultrasound scans of the carotid arteries were obtained by one investigator (GS, blinded to treatment) with high-resolution equipment (Acuson Sequoia 512 SHA, Medisales, Los Alamitos, CA); mean values of three cIMT measurements on the left and three on the right were averaged.¹⁷

### Body Composition & Abdominal Fat Partitioning

Body composition was assessed by dual X-ray absorptiometry (DXA) with a Lunar Prodigy and Lunar software (version 3.4/3.5, Lunar Corp, WI).¹⁷

Subcutaneous and visceral partitioning of abdominal fat was assessed by magnetic resonance imaging (MRI) using a multiple-slice MRI 1.5 Tesla scan (Signa LX Echo Speed Plus Excite, General Electric, Milwaukee, WI).¹⁷ MRI was also used to assess hepatic fat (%), by comparing the intensity of the liver to that of subcutaneous fat and spleen, assuming that the latter organ is fat free. Scans were performed by one operator, and images were analysed by one radiologist (LdR), both of whom were blinded to treatment.

### Assays

Serum glucose was measured by glucose oxidase method. Circulating insulin and SHBG were assayed by immunochemiluminiscence (IMMULITE 2000, Diagnostic Products, Los Angeles, CA). HOMA-IR was calculated as [fasting insulin in mU/L] x [fasting glucose in mg/dL]/405. CRP was measured by a highly sensitive method (Architect c8000; Abbott, Wiesbaden, Germany). HMW adiponectin was assessed by ELISA (Linco, St. Louis, MO). Circulating testosterone and androstenedione were measured by liquid chromatography - tandem mass spectrometry, using an approach based on a described method.¹⁹ Briefly, 50 µL of sample were mixed with 20 µL of an ISTD mixture (testosterone-d3 5 ng/mL) and 150 µL of acetonitrile for protein precipitation. Samples were centrifuged and after the evaporation of the supernatant (40 °C under nitrogen stream), 1 mL of water was added. The mixture was extracted with 3 mL of terbutyl methylether and the organic layer was separated and evaporated to dryness. The extracts were reconstituted with 100 µL of a mixture of deionized water:methanol (1:1, v/v) and 10 µL were injected into the liquid chromatography - tandem mass spectrometry system (Acquity UPLC coupled to a Xevo triple quadrupole mass spectrometer, Waters). The liquid chromatography separation was performed using a gradient program; the percentage of organic solvent was linearly changed as follows: 0 min, 70 %; 0.5 min, 70 %; 3 min, 95 %; 3.5 min, 95 %; 3.6 min, 95 %; 5 min, 70 %. Androgens were determined by a Selected Reaction Monitoring method by acquiring two transitions for each analyte (289 → 97 and 289 → 109 for testosterone, 287 → 97 and 287 → 109 for androstenedione, and 292 → 97 and 292 → 109 for internal standard). Quantitative data were obtained with both transitions. Solvent standards at five concentration levels were used for quantification purposes. MassLynx software was used for data management. Free indices of testosterone and androstenedione were calculated as 100x the ratio of the circulating androgen concentration (in nmol/L) over that of sex hormone binding globulin (SHBG, in nmol/L). In the absence of normal values (with these methods, in this age range, and in this ethnic population), free androgen Z-scores were derived from results obtained with the same methods in healthy girls of similar age and ethnic background, and with regular menses (Table 2). The relative homogeneity of this indicative population led to relatively small standard deviations, and these should be taken into account when interpreting the relatively elevated Z-score deviations in the present study population.

### Ovulation

Ovulations were inferred by combining evidence from menstrual diaries and salivary progesterone measurements. Progesterone concentrations were measured in saliva obtained during the second and fourth trimesters of the post-treatment year (study months 15-18 and 21-24, in Table 1). Saliva samples were collected once weekly (on the same weekday) for 12 consecutive weeks within each trimester, for a total of 24 samples per patient. The samples (approximately 5 mL each) were delivered after a mouth rinse with clear water and prior to food intake, by spitting into a plastic collector. The use of salivation-stimulating devices was avoided. Each collector was labelled (name and date) before storage in the patient's home freezer. After completion of each 12-week saliva collection, the saliva samples were brought to the hospital in isothermal bags, along with a menstrual diary that was kept by each girl and that spanned 14 weeks (including 2 weeks after the 12 weeks of saliva collection). Upon receipt, samples were thawed and transferred into polypropylene tubes, centrifuged for 15 min at 3000 rpm to remove cellular debris, and then stored at -20°C for up to 3 months until assay. Saliva samples were defrosted and centrifuged again on the day of progesterone measurement by ELISA (Novatec, Immundiagnostica, Dietzenbach, Germany); intra- and inter-assay coefficients of variation were 5.5% and 6.0%, and the detection limit was 3.8 pg/mL. Candidate ovulations were identified by using a lower progesterone cut-off of 99 pg/mL, as recommended by the assay manufacturer. Candidate ovulations were confirmed, when the menstrual diary indicated that menses did not start up to 2 weeks prior to obtaining the concerned saliva sample, and did start within 2 weeks thereafter. All the ovulations that were confirmed in this two-step process, proved to be associated with salivary progesterone concentrations >150 pg/mL; conversely, none of the candidate ovulations with concentrations >150 pg/mL was infirmed by the menstrual pattern.

### Statistics

Analyses were performed with SPSS 22.0 (SPSS, Chicago, IL). Longitudinal changes in quantitative variables between groups were compared by repeated-measures general linear model. Differences in longitudinal changes between groups were tested by the interaction term among between- and within-subjects effects. The between-group difference in ovulation numbers was sought by Mann-Witney U test, and that in normovulatory fractions by Fisher Exact test. Associations between quantitative variables were sought by correlation analyses. P<0.05 was considered statistically significant.

### Results

### Study Completion, Treatment Adherence, Side Effects

Study completion rate was 94% (one post-treatment drop-out in each group). Treatment adherence was estimated at >97%, as judged by history and pill counts. Noteworthy side effects were spotting in the first month (17% of OC girls), and occasional/mild abdominal discomfort in the first month (11% of SPIOMET girls).

### Primary Outcome: Post-Treatment Ovulation

SPIOMET treatment was followed by a 2.5-fold higher ovulation rate, and by a 6-fold higher fraction of normovulatory girls than OC (Table 2). The absolute risk of oligo-anovulation was 65% lower after SPIOMET than OC (95% confidence interval, 40-89%). The number-needed-to-treat with SPIOMET to observe one additional PCOS girl with normovulation was 2 (95% confidence interval, 1.1-2.5).

Post-treatment ovulation rates associated to on-treatment changes of hepatic fat, and to post- *versus* pre-treatment changes of free testosteronaemia (Figure 1), also after adjustments for changes in BMI and total fat mass.

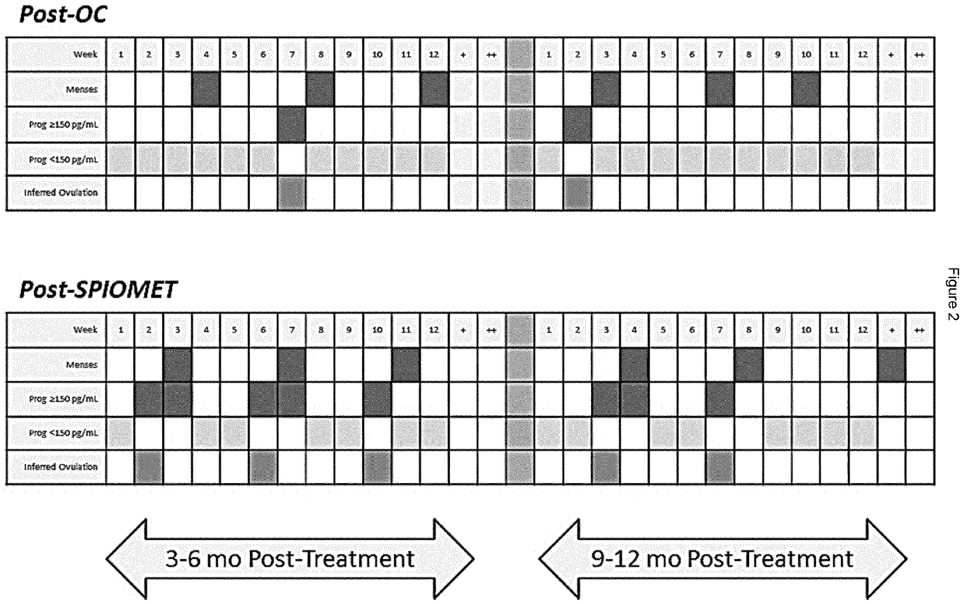

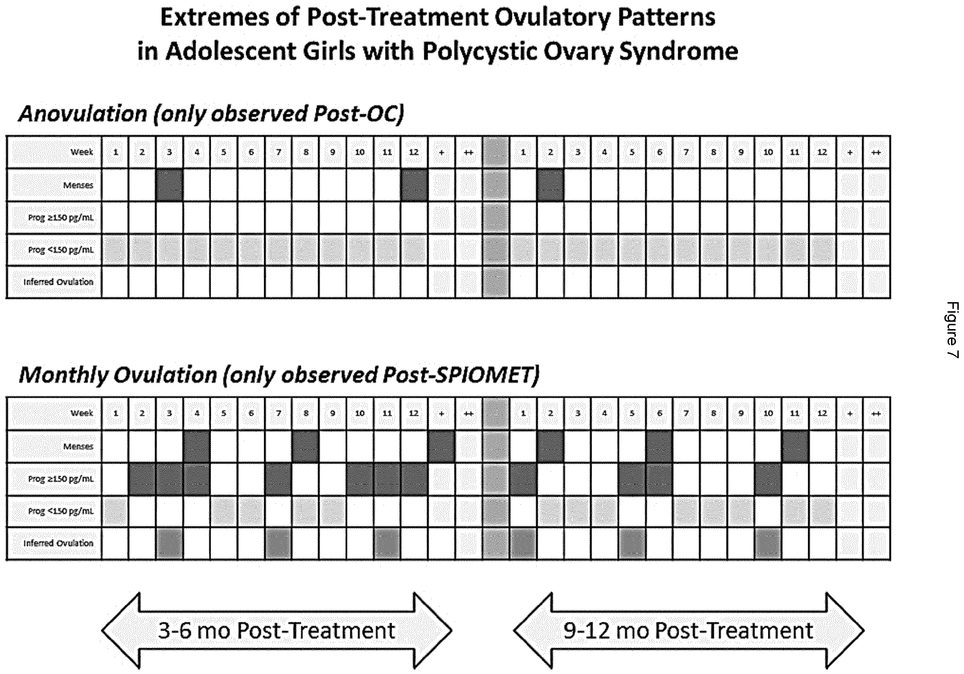

Representative patterns of menstrual history and salivary progesterone (Figure 2) illustrate that girls with similar menstrual histories after OC or SPIOMET may have markedly different ovulation rates. Low ovulation numbers (0 or 1 over 12+12 weeks) were only noted post-OC [35% of these girls], and high numbers (5 or 6) were only noted post-SPIOMET [59% of those girls] (Figure 7).

### Secondary Outcomes

SPIOMET treatment was accompanied by a spectrum of normalising effects, including on hepatic fat (Figure 3), visceral fat, waist circumference, and on circulating insulin, CRP and HMW adiponectin (Table 3). Hepatic and visceral fat, as well as glucose-stimulated insulinaemia remained more normal post-SPIOMET than post-OC (Table 4). Hyperandrogenaemia normalised more slowly during SPIOMET than OC treatment, but rebounded also more slowly post-SPIOMET (Figure 3). BMI, lean mass, and abdominal subcutaneous fat mass remained stable in both groups throughout the study.

### Conclusion

Randomised treatments for adolescent PCOS girls were followed by markedly different ovulation rates. More on-treatment loss of hepatic fat excess associated to more normal post-treatment ovulation rates.

The sequential findings in the present study corroborate a concept wherein hepato-visceral fat and/or hyperinsulinaemia are key PCOS drivers^{7,8,9} that have concerted actions at multiple levels, including an upregulation of gonadotropin secretion at the neuroendocrine level,²⁰ and an upregulation of androgen release at the ovarian level.²¹

Since the SPIOMET-associated normalisations of hepatic and visceral fat occurred in the absence of detectable changes in body weight, lean mass or total fat, they may have been elicited by a shift of fat from central/ectopic depots (liver and viscera) towards peripheral/eutopic depots (subcutaneous adipose tissue), thereby conceivably reducing any co-morbidities linked to ectopic fat storage.^{22,23}

More on-treatment loss of central-fat excess, in particular liver fat, was followed by more post-treatment ovulations. Oligo-anovulation in PCOS may thus be a mirror image of oligo-anovulation in elite athletes, which has long been linked to a central-fat deficit.²⁴ The time may have come to update the concept of "The Right Weight for Ovulation"²⁵ into a concept of "The Right Amount of Hepato-Visceral Fat for Ovulation", wherein a U-shaped curve depicts the relationship between the amount of hepato-visceral fat (along the X-axis) and the prevalence of oligo-anovulation (along the Y-axis).⁷ Most PCOS women with LH elevation, androgen excess and anovulation may not have an ovarian disorder but may rather be in an endocrine mode that is triggered beyond an individual threshold of hepato-visceral fat, and that is evolutionarily conserved as a feedback mechanism that reduces central fatness,²⁶ and as a protective mechanism that prevents high-risk pregnancies.²⁷ The delay of adolescent PCOS by early metformin treatment (age 8-12 years) associated also to the combined reduction of hepatic and visceral fat excess.^{28,29} Evolutionary speculations and ontogenic evidence thus concord to suggest that PCOS is not an ovarian syndrome, but rather a pseudo-ovarian central obesity sequence (pcos).

The present results uncovered an association between hepatic fat and ovulatory function (Figure 1), independently of body weight, lean mass, total fat mass, and abdominal subcutaneous fat mass. Although the present findings were mostly obtained in non-obese adolescents with PCOS and with a stable body weight, they may nevertheless offer an explanation as to why a healthier lifestyle and/or weight loss can be so effective to improve spontaneous ovulatory function (or responsiveness to ovulation induction) in obese adolescents and women with PCOS: the liver appears to be the first depot to lose fat upon the initiation of a healthier lifestyle and/or weight loss, and viscera appear to be next.^{30,31} Indeed, in obese persons on a low-calorie diet, an approximate weight loss of 10% is accompanied by approximate reductions of 60% in hepatic fat, 20% in visceral fat, and only 10% in subcutaneous fat.³¹

The recognition of a key role for hepato-visceral fat in the phenotype of PCOS offers an explanation as to why non-obese adolescents with PCOS tend to have a rather low birth weight:^{7,9} girls with a lower birth weight tend to have more hepatic and visceral fat (independently of BMI), and this from early childhood onwards.^{32,33}

In conclusion, SPIOMET normalises post-treatment ovulation rates of adolescent girls with PCOS more than OC does. Refocusing PCOS treatment towards early reduction of ectopic fat, in particular liver fat, may prevent part of subsequent oligo-anovulatory subfertility.

### Example 2: Pharmaceutical tablet

For the manufacture of the pharmaceutical tablet, active substances spironolactone, metformin and pioglitazone are mixed with polyvinylpirrolidone. A low shear wet granulation is performed and the granules are dried and milled. Remaining excipients (sodium croscarmellose, microcrystalline cellulose, magnesium stearate and polyvinyl alcohol) are blended with the milled granules and altogether mixed with lubricant polyvinyl alcohol. The blend is finally compressed into tablets having the following composition.

**The resulting tablet core contained:**

| **Tablet core** | **Quantity / mg** | **Function** |
|---|---|---|
| **Core** | | |
| spironolactone | 50 | Active principle ingredient |
| pioglitazone | 7.5 | Active principle ingredient |
| metformin | 850 | Active principle ingredient |
| Polyvinylpirrolidone | 55 | Binder |
| Sodium croscarmellose | 30 | Diluent |
| Microcrystalline cellulose | 70 | Disgregant |
| Magnesium stearate | 10 | Lubricant |
| Polyvinyl alcohol | 26,5 | Film forming polymer |

In one embodiment the tablet was coated with a film coating.

### Items of the invention

1. A pharmaceutical composition comprising spironolactone, pioglitazone and metformin for use in treating a condition that benefits from the reduction of hepatic and/or visceral fat.
2. The pharmaceutical composition according to item 1 for use in treating a condition that benefits from the reduction of hepatic and/or visceral fat in adolescent girls or women of childbearing age.
3. The pharmaceutical composition according to items 1 or 2 wherein the condition is selected from the group comprising polycystic ovary syndrome, metabolically obese normal weight syndrome, metabolic syndrome, obesity or being overweight.
4. A pharmaceutical composition comprising spironolactone, pioglitazone and metformin for use in the prevention or treatment of polycystic ovary syndrome in adolescent girls or women of childbearing age.
5. A pharmaceutical composition combining spironolactone, pioglitazone and metformin for use in the prevention or treatment of low ovulation rate associated with liver steatosis in adolescent girls or women of childbearing age.
6. The pharmaceutical composition according to items 1 to 5 wherein each of spironolactone, pioglitazone and metformin are administered sequentially in separate single delivery forms.
7. The pharmaceutical composition according to items 1 to 5 wherein two of spironolactone, pioglitazone and metformin are administered in a single delivery form and the remaining compound is administered in a separate delivery form.
8. The pharmaceutical composition according to items 6 or 7 wherein the single delivery forms are administered at the same time or within 5 minutes to 1 hour of each other, preferably within 15 minutes to 30 minutes of each other.
9. The pharmaceutical composition according to item 5 wherein the liver steatosis is not associated with overweight or obesity.
10. The pharmaceutical composition according to items 5 or 9 wherein the adolescent girls or women of childbearing age exhibit androgen excess.
11. The pharmaceutical composition according to items 5, 9 or 10 wherein the adolescent girls or women of childbearing age exhibit hyperinsulinemia, low adiponectin, high C-reactive protein levels and/or high gonadotropin levels.
12. The pharmaceutical composition according to items 5, 9, 10 or 11 wherein the adolescent girls or women of childbearing age exhibit visceral fat excess and/or Dyslipidemia.
13. The pharmaceutical composition according to any of the items 2 to 12 wherein the adolescent girls or women of childbearing age use an oral or non-oral contraceptive.
14. The pharmaceutical composition according to item 13 wherein said non-oral contraceptive is an intra-uterine contraceptive.
15. The pharmaceutical composition according to any of the items 1 to 14 wherein a daily dose of said pharmaceutical composition comprises between 25 and 100 mg spironolactone.
16. The pharmaceutical composition according to any of the items 1 to 15 wherein a daily dose of said composition comprises between 5 and 15 mg pioglitazone.
17. The pharmaceutical composition according to any of the items 1 to 16 wherein a daily dose of said composition comprises between 500 and 1500 mg metformin.
18. A method for treating a condition that benefits from the reduction of hepatic and/or visceral fat said method comprising the combined administration of spironolactone, pioglitazone and metformin.
19. The method according to item 18 for treating a condition that benefits from the reduction of hepatic and/or visceral fat in adolescent girls or women of childbearing age.
20. The method according to items 18 or 19 wherein the condition is selected from the group comprising polycystic ovary syndrome, metabolically obese normal weight syndrome, metabolic syndrome, obesity or being overweight.
21. A method for preventing or treating polycystic ovary syndrome in adolescent girls or women of childbearing age said method comprising the combined administration of spironolactone, pioglitazone and metformin.
22. A method for preventing or treating low ovulation rate associated with liver steatosis in adolescent girls or women of childbearing age said method comprising the combined administration of spironolactone, pioglitazone and metformin.
23. The method according to items 18 to 22 wherein each of spironolactone, pioglitazone and metformin are administered sequentially in separate single delivery forms.
24. The method according to items 18 to 22 wherein two of spironolactone, pioglitazone and metformin are administered in a single delivery form and the remaining compound is administered in a separate delivery form.
25. The method according to items 23 or 24 wherein the single delivery forms are administered at the same time or within 5 minutes to 1 hour of each other, preferably within 15 minutes to 30 minutes of each other.
26. The method according to item 22 wherein the liver steatosis is not associated with overweight or obesity.
27. The method according to items 22 or 26 wherein the adolescent girls or women of childbearing age exhibit androgen excess.
28. The method according to items 22, 26 or 27 wherein the adolescent girls or women of childbearing age exhibit hyperinsulinemia, low adiponectin, high C-reactive protein levels and/or high gonadotropin levels.
29. The method according to items 22, 26, 27, or 28 wherein the adolescent girls or women of childbearing age exhibit visceral fat excess and/or Dyslipidemia.
30. The method according to any of the items 18 to 29 wherein said method comprises the daily administration of between 25 and 100 mg spironolactone.
31. The method according to any of the items 18 to 30 wherein said method comprises the daily administration of between 5 and 15 mg pioglitazone.
32. The method according to any of the items 18 to 31 wherein said method comprises the daily administration of between 500 and 1500 mg metformin.
33. The method according to any of the items 19 to 32 wherein the adolescent girls or women of childbearing age use an oral or non-oral contraceptive.
34. The method according to item 33 wherein said non-oral contraceptive is an intra-uterine contraceptive.
35. The method according to item 33 or 34 wherein said method further comprises ending the non-oral contraceptive following the termination of said combined administration of spironolactone, pioglitazone and metformin in order to allow for conception.
36. A pharmaceutical composition wherein said pharmaceutical composition comprises between 25 and 100 mg spironolactone and/or between 5 and 15 mg pioglitazone and/or between 500 and 1500 mg metformin.
37. The pharmaceutical composition according to item 36 wherein said pharmaceutical composition comprises between 25 and 100 mg spironolactone and between 5 and 15 mg pioglitazone.
38. The pharmaceutical composition according to item 36 wherein a daily dose of said pharmaceutical composition comprises between 25 and 100 mg spironolactone, between 5 and 15 mg pioglitazone, and between 500 and 1500 mg metformin.
39. The pharmaceutical composition according to item 38 wherein a daily dose of said pharmaceutical composition comprises 50 mg spironolactone, 7.5 mg pioglitazone, and 850 mg metformin.
40. The pharmaceutical composition according to items 36 to 39 wherein said pharmaceutical composition is in an oral delivery form.
41. The pharmaceutical composition according to item 40 wherein said oral delivery form is a tablet or capsule.

### References

1. Legro RS, Arslanian SA, Ehrmann DA, Hoeger KM, Murad MH, Pasquali R, Welt CK; Endocrine Society. Diagnosis and treatment of polycystic ovary syndrome: an Endocrine Society clinical practice guideline. J Clin Endocrinol Metab 2013;98:4565-92.
2. Dumesic DA, Oberfield SE, Stener-Victorin E, Marshall JC, Laven JS, Legro RS. Scientific statement on the diagnostic criteria, epidemiology, pathophysiology, and molecular genetics of polycystic ovary syndrome. Endocr Rev 2015;36:487-525.
3. Kuchenbecker WK, Groen H, van Asselt SJ, Bolster JH, Zwerver J, Slart RH, Vd Jagt EJ, Muller Kobold AC, Wolffenbuttel BH, Land JA, Hoek A. In women with polycystic ovary syndrome and obesity, loss of intra-abdominal fat is associated with resumption of ovulation. Hum Reprod 2011;26:2505-12.
4. Jones H, Sprung VS, Pugh CJ, Daousi C, Irwin A, Aziz N, Adams VL, Thomas EL, Bell JD, Kemp GJ, Cuthbertson DJ. Polycystic ovary syndrome with hyperandrogenism is characterised by an increased risk of hepatic steatosis compared to nonhyperandrogenic PCOS phenotypes and healthy controls, independent of obesity and insulin resistance. J Clin Endocrinol Metab 2012;97:3709-16.
5. Göbl CS, Ott J, Bozkurt L, Feichtinger M, Rehmann V, Cserjan A, Heinisch M, Steinbrecher H, JustKukurova I, Tuskova R, Leutner M, Vytiska-Binstorfer E, Kurz C, Weghofer A, Tura A, Egarter C, Kautzky-Willer A. To assess the association between glucose metabolism and ectopic lipid content in different clinical classifications of PCOS. PLoS One 2016;11:e0160571.
6. Witchel SF, Oberfield S, Rosenfield RL, Codner E, Bonny A, Ibáñez L, Pena A, Horikawa R, Gomez-Lobo V, Joel D, Tfayli H, Arslanian S, Dabadghao P, Garcia Rudaz C, Lee PA. The diagnosis of polycystic ovary syndrome during adolescence. Horm Res Paediatr 2015 Apr 1 [Epub].
7. Ibáñez L, Ong KK, Lopez-Bermejo A, Dunger DB, de Zegher F. Hyperinsulinemic androgen excess in adolescent girls. Nat Rev Endocrinol 2014;100:499-508.
8. de Zegher F, Ibáñez L. Early origins of polycystic ovary syndrome: hypotheses may change without notice. J Clin Endocrinol Metab 2009; 94:3682-5.
9. de Zegher F, Lopez-Bermejo A, Ibáñez L. Adipose tissue expandability and the early origins of PCOS. Trends Endocrinol Metab 2009; 20:418-23.
10. Azziz R, Carmina E, Chen Z, Dunaif A, Laven JS, Legro RS, Lizneva D, Natterson-Horowitz B, Teede HJ, Yildiz BO. Polycystic ovary syndrome. Nat Rev Dis Primers 2016;2:16057.
11. Lass N, Kleber M, Winkel K, Wunsch R, Reinehr T. Effect of lifestyle intervention on features of polycystic ovarian syndrome, metabolic syndrome, and intima-media thickness in obese adolescent girls. J Clin Endocrinol Metab 2011; 96:3533-40.
12. Hecht Baldauff N, Arslanian S. Optimal management of polycystic ovary syndrome in adolescence. Arch Dis Child 2015;100:1076-83.
13. Rosenfield RL. The diagnosis of polycystic ovary syndrome in adolescents. Pediatrics 2015;136:1154-65.
14. Bonny AE, Appelbaum H, Connor EL, Cromer B, DiVasta A, Gomez-Lobo V, Harel Z, Huppert J, Sucato G; NASPAG Research Committee. Clinical variability in approaches to polycystic ovary syndrome. J Pediatr Adolesc Gynecol 2012;25:259-61.
15. Ibáñez L, Valls C, Potau N, Marcos MV, de Zegher F. Sensitization to insulin in adolescent girls to normalise hirsutism, hyperandrogenism, oligomenorrhea, dyslipidaemia and hyperinsulinism after precocious pubarche. J Clin Endocrinol Metab 2000; 85:3526-30.
16. Ibáñez L, de Zegher F. Low-dose flutamide-metformin therapy for hyperinsulinemic hyperandrogenism in non-obese adolescents and women. Hum Reprod Update 2006; 12:243-52.
17. Ibáñez L, Diaz M, Sebastiani G, Marcos MV, Lopez-Bermejo A, de Zegher F. Oral contraception vs insulin sensitization for 18 months in nonobese adolescents with androgen excess: posttreatment differences in C-reactive protein, intima-media thickness, visceral adiposity, insulin sensitivity, and menstrual regularity. J Clin Endocrinol Metab 2013; 98:E902-7.
18. Goodman NF, Cobin RH, Futterweit W, Glueck JS, Legro RS, Carmina E. American Association of Clinical Endocrinologists, American College of Endocrinology, and Androgen Excess and PCOS Society disease state and clinical review: guide to the best practices in the evaluation and treatment of Polycystic Ovary Syndrome - Part 1. Endocr Pract 2015;21:1291-300.
19. Marcos J, Renau N, Casals G, Segura J, Ventura R, Pozo OJ. Investigation of endogenous corticosteroids profiles in human urine based on liquid chromatography tandem mass spectrometry. Anal Chim Acta 2014; 812:92-104.
20. George JT, Kakkar R, Marshall J, Scott ML, Finkelman RD, Ho TW, Veldhuis J, Skorupskaite K, Anderson RA, McIntosh S, Webber L. Neurokinin B receptor antagonism in women with Polycystic Ovary Syndrome: a randomized, placebo-controlled trial. J Clin Endocrinol Metab 2016 Jul 26:jc20161202. [Epub ahead of print]
21. McAllister JM, Legro RS, Modi BP, Strauss JF 3rd. Functional genomics of PCOS: from GWAS to molecular mechanisms. Trends Endocrinol Metab 2015;26:118-24.
22. Shulman GI. Ectopic fat in insulin resistance, dyslipidaemia, and cardiometabolic disease. N Engl J Med 2014;371:2237-8.
23. Yki-Järvinen H. Non-alcoholic fatty liver disease as a cause and a consequence of metabolic syndrome. Lancet Diabetes Endocrinol 2014;2:901-10.
24. Frisch RE, Snow RC, Johnson LA, Gerard B, Barbieri R, Rosen B. Magnetic resonance imaging of overall and regional body fat, estrogen metabolism, and ovulation of athletes compared to controls. J Clin Endocrinol Metab 1993;77:471-7.
25. Frisch RE. The right weight: body fat, menarche and ovulation. Baillieres Clin Obstet Gynaecol 1990;4:419-39.
26. Dumesic DA, Abbott DH, Eisner JR, Herrmann RR, Reed JE, Welch TJ, Jensen MD. Pituitary desensitization to gonadotropin-releasing hormone increases abdominal adiposity in hyperandrogenic anovulatory women. Fertil Steril 1998;70:94-101.
27. Palomba S, de Wilde MA, Falbo A, Koster MP, La Sala GB, Fauser BC. Pregnancy complications in women with polycystic ovary syndrome. Hum Reprod Update 2015;21:575-92.
28. Ibáñez L, Lopez-Bermejo A, Diaz M, Marcos MV, de Zegher F. Pubertal metformin therapy to reduce total, visceral, and hepatic adiposity. J Pediatr 2010;156:98-102.
29. Ibáñez L, Lopez-Bermejo A, Diaz M, Marcos MV, de Zegher F. Early metformin therapy (age 8-12 years) in girls with precocious pubarche to reduce hirsutism, androgen excess, and oligomenorrhea in adolescence. J Clin Endocrinol Metab 2011;96:E1262-7.
30. Fonvig CE, Chabanova E, Ohrt JD, Nielsen LA, Pedersen O, Hansen T, Thomsen HS, Holm JC. Multidisciplinary care of obese children and adolescents for one year reduces ectopic fat content in liver and skeletal muscle. BMC Pediatr 2015;15:196.
31. Vogt LJ, Steveling A, Meffert PJ, Kromrey ML, Kessler R, Hosten N, Krüger J, Gärtner S, Aghdassi AA, Mayerle J, Lerch MM, Kühn JP. Magnetic resonance imaging of changes in abdominal compartments in obese diabetics during a low-calorie weight-loss program. PLoS One 2016;11:e0153595.
32. Ibáñez L, Lopez-Bermejo A, Suárez L, Marcos MV, Diaz M, de Zegher F. Visceral adiposity without overweight in children born small for gestational age. J Clin Endocrinol Metab 2008;93:2079-83.
33. Sebastiani G, Diaz M, Bassols J, Aragonés G, Lopez-Bermejo A, de Zegher F, Ibáñez L. The sequence of prenatal growth restraint and post-natal catch-up growth leads to a thicker intima-media and more pre-peritoneal and hepatic fat by age 3-6 years. Pediatr Obes 2016;11:251-7.
34. Ibáñez L, de Zegher F, Potau N. Anovulation after precocious pubarche: early markers and time course in adolescence. J Clin Endocrinol Metab 1999;84:2691-5.
35. Legro RS, Dodson WC, Kris-Etherton PM, Kunselman AR, Stetter CM, Williams NI, Gnatuk CL, Estes SJ, Fleming J, Allison KC, Sarwer DB, Coutifaris C, Dokras A. Randomized controlled trial of preconception interventions in infertile women with polycystic ovary syndrome. J Clin Endocrinol Metab 2015;100:4048-58.
36. Legro RS, Dodson WC, Kunselman AR, Stetter CM, Kris-Etherton PM, Williams NI, Gnatuk CL, Estes SJ, Allison KC, Sarwer DB, Diamond MP, Schlaff WD, Casson PR, Christman GM, Barnhart KT, Bates GW, Usadi R, Lucidi S, Baker V, Zhang H, Eisenberg E, Coutifaris C, Dokras A. Benefit of delayed fertility therapy with preconception weight loss over immediate therapy in obese women with PCOS. J Clin Endocrinol Metab 2016;101:2658-66.
37. De Souza LR, Berger H, Retnakaran R, Maguire JL, Nathens AB, Connelly PW, Ray JG. First-trimester maternal abdominal adiposity predicts dysglycaemia and gestational diabetes mellitus in midpregnancy. Diabetes Care 2016;39:61-4.

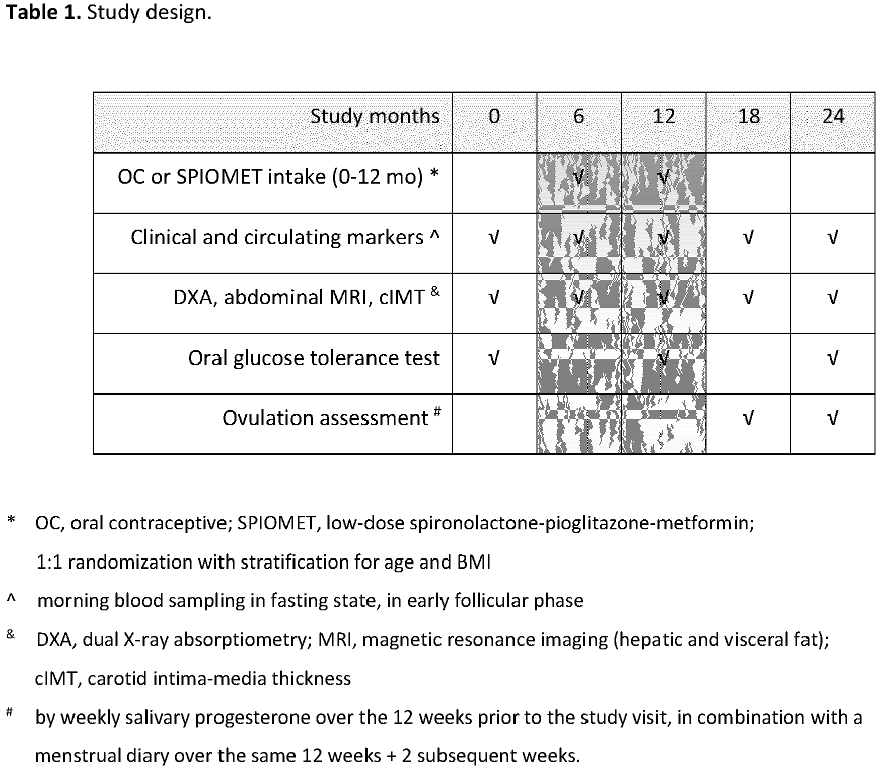

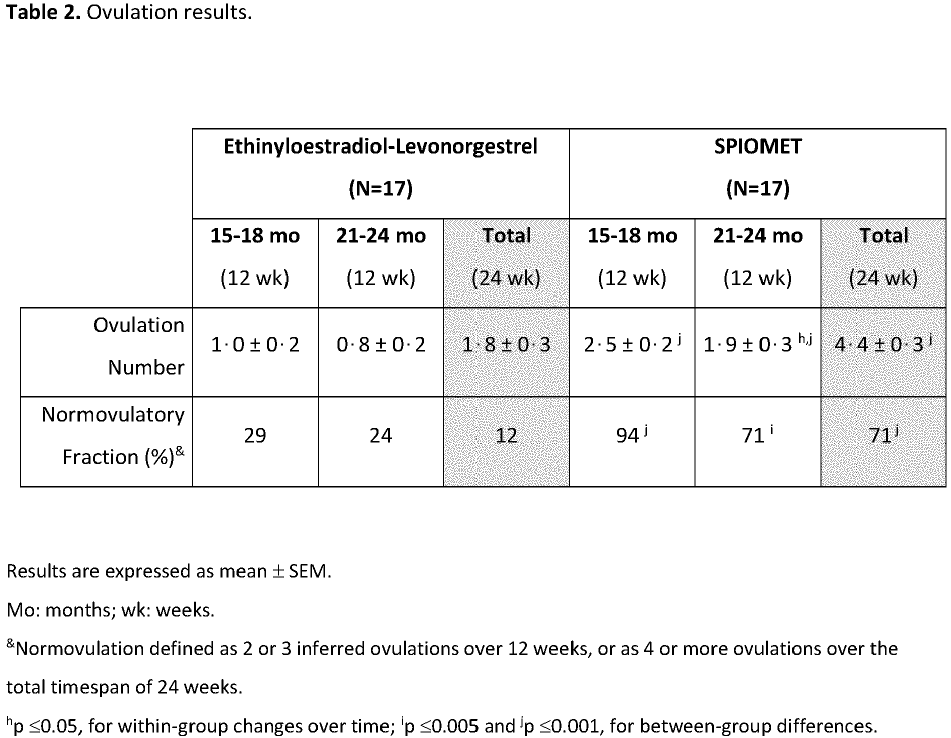

**Table 3. On-treatment results (0-12 months).**

| | **Ethinyloestradiol-Levonorcestrel (N=18)** | | | | **SPIOMET (N=18)** | | | |
|---|---|---|---|---|---|---|---|---|
| | **Start**^{a} | **6 mo** | **12 mo** | *Δ **0-12 mo*** | **Start^{a}** | **6 mo** | **12 mo** | ***Δ 0-12 mo*** |
| Birthweight Z-score | -0.5 ± 0.2 | | | | -0.4 ± 0.3 | | | |
| Age at Menarche (yr) | 11.7 ± 0.2 | | | | 11.7 ± 0.2 | | | |
| Age (yr) | 15.9 ± 0.3 | | | | 15.8 ± 0.3 | | | |
| BMI (Kg/m²) | 24.0 ± 0.8 | | | | 23.4 ± 0.7 | | | |
| BMI Z-score | 0.8 ± 0.2 | 0.7 ± 0.2 | 0.8 ± 0.3 | *0.0 ± 0.3* | 0.6 ± 0.2 | 0.5 ± 0.2 | 0.5 ± 0.2 | *-0.1 ± 0.1* |
| Waist Circumference (cm) | 75 ± 2 | 76 ± 2 | 76 ± 2 | *1 ± 1* | 76 ± 2 | 73 ± 1 | 73 ± 1^{d} | *-4 ± 1^{g}* |
| Endogenous Regular Menses (%) | 33 | 0^{c^} | 0^{c^} | *-33* | 28 | 89^{d} | 94 ^{d} | *66^{g}* |
| Hirsutism Score | 17 ± 1 | 14 ± 1^{d} | 13 ± 1^{d} | *-3 ± 1* | 15 ± 1 | 11 ± 1^{d} | 9 ± 1^{d} | *-6 ± 1* |
| Acne Score | 1.9 ± 0.2 | 1.4 ± 0.1^{d} | 1.3 ± 0.1^{d} | *-0.6 ± 0.2* | 2.1 ± 0.2 | 1.2 ± 0.1^{d} | 1.0 ± 0.1^{d} | *-1.1 ± 0.2* |
| SHBG (nmol/L)* | 30 ± 3 | 65 ± 8^{d} | 61 ± 6^{d} | *31 ± 6* | 28 ± 2 | 34 ± 3^{b} | 34 ± 3^{b} | *6 ± 3^{g}* |
| Testosterone (nmol/L)* | 1.2 ± 0.1 | 0.7 ± 0.1^{c} | 0.5 ± 0.1^{d} | *-0.7 ± 0.1* | 1.2 ± 0.1 | 1.0 ± 0.1 | 0.6 ± 0.1^{d} | *-0.6 ± 0.1* |
| Androstenedione (nmol/L)* | 5.3 ± 0.4 | 3.5 ± 0.3^{d} | 2.4 ± 0.2^{d} | *-2.9 ± 0.4* | 6.3 ± 0.5 | 4.4 ± 0.3^{c} | 3.0 ± 0.2^{d} | *-3.2 ± 0.5* |
| Free Testosterone Z-score | 6.8 ± 0.8 | 0.1 ± 0.4^{d} | -0.2 ± 0.3^{d} | *-7.0 ± 0.8* | 7.9 ± 1.3 | 5.0 ± 0.9^{b} | 1.6 ± 0.5^{d} | *-6.3 ± 1.1* |
| Free Androstenedione Z-score | 5.6 ± 1.0 | 0.2 ± 0.3^{d} | -0.4 ± 0.4^{d} | *-5.9 ± 0.9* | 6.9 ± 0.7 | 3.4 ± 0.6^{d} | 1.9 ± 0.7^{d} | *- 5.0 ± 0.8* |
| Fasting Insulin (pmol/L)* | 92 ± 12 | 97 ± 9 | 106 ± 16 | *14 ± 11* | 76 ± 8 | 54 ± 7^{c} | 43 ± 6^{d} | *-33 ± 8^{g}* |
| HOMA-IR* | 2.9 ± 0.4 | 3.0 ± 0.3 | 3.2 ± 0-5 | *0.3 ± 0.4* | 2.3 ± 0.3 | 1.7 ± 0.2^{b} | 1.2 ± 0.2^{d} | *-1.0 ± 0.3^{f}* |
| **oGTT** Mean Glycaemia Z-score | 0.1 ± 0.1 | | 0.2 ± 0.1 | *0.1 ± 0.1* | 0.3 ± 0.1 | | 0.2 ± 0.1 | *-0.1 ± 0.1* |
| Mean Insulinaemia Z-score | 3.6 ± 0.6 | | 3.6 ± 0.7 | *0.0 ± 0.6* | 2.8 ± 0.5 | | 0.5 ± 0.3^{d} | *-2.3 ± 0.4^{f}* |
| ALT (IU/L) | 15 ± 1 | 17 ± 2 | 19 ± 2^{b} | *4 ± 2* | 14 ± 1 | 14 ± 1 | 15 ± 2 | *2 ± 2* |
| AST (IU/L) | 16 ± 1 | 17 ± 2 | 17 ± 1 | *1 ± 1* | 16 ± 1 | 17 ± 1 | 17 ± 1 | *1 ± 1* |
| GGT (IU/L) | 12 ± 1 | 15 ± 1^{c} | 16 ± 1^{d} | *4 ± 1* | 13 ± 1 | 11 ± 1^{b} | 11 ± 1^{b} | *-2 ± 1^{g}* |
| Triacylglycerol (mmol/L) | 0.6 ± 0.04 | 0.7 ± 0.04 | 0.6 ± 0.04 | *0.0 ± 0.04* | 0.7 ± 0.1 | 0.6 ± 0.1 | 0.5 ± 0.04^{b} | *-0.2 ± 0.04^{e}* |
| LDL-cholesterol (mmol/L) | 2.3 ± 0.1 | 2.6 ± 0.1^{b} | 2.6 ± 0.1^{b} | *0.3 ± 0.1* | 2.4 ± 0.1 | 2.2 ± 0.1 | 2.4 ± 0.1 | *0.0 ± 0.1^{e}* |
| HDL-cholesterol (mmol/L) | 1-3 ± 0.05 | 1.4 ± 0.05 | 1.3 ± 0.05 | *0.0 ± 0.02* | 1.3 ± 0.05 | 1.4 ± 0.05^{b} | 1.5 ± 0-1^{c} | *0.2 ± 0.05^{f}* |
| HMW-adiponectin (mg/L)* | 7.7 ± 0.3 | 9.2 ± 1.3 | 12.6 ± 1.5^{c} | *4.8 ± 1.6* | 9.6 ± 1.4 | 13.4 ± 1.7 | 25.4 ± 3.7^{d} | *15.6 ± 3.1^{f}* |
| C-Reactive Protein (mg/L)* | 1.4 ± 0.2 | 2.7 ± 0.4^{b} | 2.8 ± 0.6^{b} | *1.4 ± 0.7* | 1.5 ± 0.4 | 0.5 ± 0.1^{c} | 0.5 ± 0.1^{c} | *-1.0 ± 0.3^{f}* |
| Carotid IMT (mm) | .37 ± .01 | .37 ± .01 | .37 ± .01 | *.00 ± .01* | .37 ± .01 | .36 ± .01^{d} | .36 ± .01 ^{d} | *- .02 ± .01 ^{e}* |
| Systolic Blood Pressure (mmHg) | 115 ± 2 | 112 ± 3 | 114 ± 2 | *-1 ± 2* | 114 ± 2 | 111 ± 2 | 109 ± 1^{b} | *-5 ± 2* |
| Diastolic Blood Pressure (mmHg) | 72 ± 2 | 76 ± 2 | 75 ± 3 | *4 ±3* | 71 ± 2 | 73 ± 1 | 70 ± 1 | *-1 ± 2* |
| **DXA** BMD (g/cm²) | 1.17 ± 0.03 | 1.18 ± 0.03 | 1.18 ± 0-03 | *0.01 ± 0.01* | 1-17 ± 0.03 | 1.16 ± 0.03 | 1.18 ± 0-03 | *0.01 ± 0.01* |
| Lean Mass (Kg)^{#} | 35.1 ± 1.0 | 34.9 ± 1.0 | 34.8 ± 1.0 | *-0.3 ± 0.2* | 35.6 ± 1.1 | 35.7 ± 1.0 | 35.4 ± 1.0 | *-0.2 ± 0.3* |
| Fat Mass (Kg)^{#} | 20.8 ± 1.5 | 20.5 ± 1.4 | 21.1 ± 1-5 | *0.3 ± 0.7* | 20.8 ± 1.7 | 20.1 ± 1.5 | 20.7 ± 1-7 | *-0.1 ± 0.7* |
| **Abd MRI** Subc Fat (cm²) | 149 ± 18 | 145 ± 17 | 142 ± 18 | *-8 ± 10* | 155 ± 18 | 142 ± 17 | 146 ± 17 | *-10 ± 7* |
| Visceral Fat (cm²) | 43 ± 4 | 44 ± 5 | 42 ± 5 | *-1 ± 4* | 49 ± 5 | 37 ± 2^{b} | 33 ± 2^{c} | *-16 ± 5^{e}* |
| Liver Fat (%) | 17 ± 1 | 21 ± 1 | 20± 2 | *3 ± 1* | 17 ± 1 | 12 ± 1 ^{d} | 10 ± 1^{d} | *-6 ± 1^{g}* |

| | | | | | | | | |
|---|---|---|---|---|---|---|---|---|
| Values are mean ± SEM. SHBG, sex hormone-binding globulin; HOMA-IR: homeostasis model assessment insulin resistance; oGTT, oral glucose tolerance test; GGT, gamma-glutamyl transpeptidase; HMW, high molecular weight; IMT, intima-media thickness; DXA, dual X-ray absorptiometry; Abd MRI, abdominal magnetic resonance imaging; Subc, subcutaneous. ^ 100% of these girls had pseudo-menses within 28 days, following the intake of placebo pills for 7/28 days. *Indicative values in healthy adolescents (n=18; age 16.9 ± 0.3 yr; BMI 21.1 ± 0.6 Kg/m²); SHBG 59 ± 5 nmol/L; testosterone 0.7 ± 0.1 nmol/L; androstenedione 3.4 ± 0.2 nmol/L; fasting insulin 21.0 ± 2.1 pmol/L; HOMA-IR 0.7 ± 0.1; GGT 12 ±1 IU/L; HMW-adiponectin 12.7 ± 1.6 mg/L; CRP 0.7 ± 0.2 mg/L. "Indicative DXA values in healthy adolescents, matched for age and height (n=41): lean mass 35.1 ± 1.0 Kg; fat mass 17.6 ± 1.4 Kg indicative MRI values in healthy adolescents (n=15; age 15.7 ± 0.3 yr; BMI 22.1 ± 0.8 Kg/m²): subc fat 97 ± 11 cm²; visceral fat 32 ± 2 cm²; hepatic fat 12 ± 1 %. ^{a}no significant differences between randomised subgroups at start. ^{b}p ≤0.05, ^{c}p≤0.01 and ^{d}p ≤0.001 for within-subgroup changes (Δ) from start. ^{e}p ≤0.05, ^{f}p ≤0.01, ^{g}p ≤0.001 for between-subgroup differences in Δ 0-12 months. | | | | | | | | |

**Table 4. Post-treatment results (12-24 months) and cumulative changes (0-24 months).**

| | **Ethinyloestradiol-Levonorgestrel (N=17)** | | | | **SPIOMET (N=17)** | | | |
|---|---|---|---|---|---|---|---|---|
| | **12 mo** | **18 mo** | **24 mo** | ***Δ 0-24* mo**° | **12 mo** | **18 mo** | **24 mo** | ***Δ 0-24 mo**°* |
| Birthweight Z-score | -0.6 ± 0.2 | | | | -0.6 ± 0.2 | | | |
| Age at Menarche (yr) | 11.7 ± 0.2 | | | | 11.7 ± 0.2 | | | |
| Age (yr) | 17.0 ± 0.3 | | | | 16.7 ± 0.3 | | | |
| BMI (Kg/m²) | 23.9 ± 0.8 | | | | 22.8 ± 0.7 | | | |
| BMI Z-score | 0.8 ± 0.3 | 0.9 ± 0.3 | 0.9 ± 0.3 | *0.1 ± 0.1* | 0.4 ± 0.2 | 0.6 ± 0.2 | 0.4 ± 0.3 | *-0.1 ± 0.1* |
| Waist Circumference (cm) | 76 ± 2 | 75 ± 2 | 76 ± 2 | *1 ± 1* | 72 ± 1 ^{d} | 72 ± 1^{d} | 72 ± 1^{d} | *-3 ± 1^{f}* |
| Endogenous Regular Menses (%) | 0 ^{c^} | 53 | 59 | *24* | 94 ^{d} | 88 ^{d} | 82^{d} | *53* |
| Hirsutism Score | 13 ± 1^{d} | 13 ± 1^{c} | 13 ± 1^{d} | *-4 ± 1* | 9 ± 1^{d} | 8 ± 1^{d} | 7 ± 1^{d} | *-7 ± 1^{f}* |
| Acne Score | 1.3 ± 0.1 ^{d} | 1.8 ± 0.2 | 1-7 ± 0.2 | *-0.4 ± 0.2* | 1.0 ± 0.1^{d} | 1.0 ± 0.1^{d} | 1.1 ± 0.1^{d} | *-1.1 ± 0.2^{e}* |
| SHBG (nmol/L)* | 62 ± 6 ^{d} | 35 ± 4 | 35 ± 4 | *6 ± 4* | 34 ± 3 ^{b} | 42 ± 5^{c} | 43 ± 4^{c} | *14 ± 4* |
| Testosterone (nmol/L)* | 0.6 ± 0.1 ^{b} | 1.5 ± 0.2 | 1.6 ± 0.2 | *0.3 ± 0.2* | 0.6± 0.1 ^{d} | 1.1 ± 0.1 | 1.2 ± 0.2 | *0.1 ± 0.2* |
| Androstenedione (nmol/L)* | 2.5 ± 0.2 ^{d} | 7.5 ± 1.0^{b} | 5.7 ± 0.6 | *0.8 ± 0.9* | 3.0 ± 0.2 ^{d} | 4.5 ± 0.3^{b} | 5.3 ± 0.6 | -*0.5 ± 0-5* |
| Free Testosterone Z-score | -0.3 ± 0.3^{d} | 9.7 ± 2.3 | 8.7 ± 1.7 | *1.8 ± 1.9* | 1.6 ± 0.5^{d} | 3.9 ± 0.9^{c} | 3.9 ± 0.7^{b} | *-3.7 ± 1.5^{e}* |
| Free Androstenedione Z-score | -0.4 ± 0.4^{d} | 9.4 ± 3.4 | 5.3 ± 1.1 | *-04 ± 1.4* | 2.0 ± 0.7^{d} | 2.7 ± 0.7^{d} | 3.0 ± 0.7^{d} | *-3.7 ± 0.9 ^{e}* |
| Fasting Insulin (pmol/L)* | 109 ± 16 | 91 ± 11 | 75 ± 10 | *-18 ± 10* | 43 ± 6^{d} | 53 ± 7 ^{b} | 46 ± 7^{c} | *-24 ± 8* |
| HOMA-IR* | 3.3 ± 0.5 | 3.1 ± 0.4 | 2.2 ± 0.3 ^{c} | *-0.8 ± 0.2* | 1.2 ± 0.2 ^{d} | 1.6 ± 0.2 ^{b} | 1.3 ± 0.2 ^{c} | *-0.8 ± 0.2* |
| **oGTT** Mean Glycaemia Z-score | 0.2 ± 0.1 | | 0.2 ± 0.1 | *0.0 ± 0.1* | 0.1 ± 0.1 | | 0.2 ± 0.1 | *-0.1 ± 0.1* |
| Mean Insulinaemia Z-score | 3.8 ± 0.7 | | 2.8 ± 0.5 | *-0.9 ± 0.6* | 0.3 ± 0.3 ^{d} | | 0.5 ± 0.3 ^{d} | *-2.2 ± 0.4^{e}* |
| ALT (IU/L) | 18 ± 2 | 18 ± 3 | 14 ± 2 | *-1 ± 2* | 15 ± 1 | 13 ± 1 | 14 ± 1 | *1 ± 2* |
| AST (IU/L) | 16 ± 1 | 16 ± 1 | 15 ± 1 | *0 ± 1* | 17 ± 1 | 17 ± 2 | 15 ± 2 | -*1 ± 1* |
| GGT (IU/L) | 17 ± 1^{d} | 14 ± 1 | 13 ± 1 | *1 ± 1* | 11 ± 1^{b} | 12 ± 1 | 12 ± 1 | *0 ± 1* |
| Triacylglycerol (mmol/L) | 0.7 ± 0.04 | 0.5 ± 0.02 | 0.5 ± 0.02 | *-0.1 ± 0.04* | 0.6 ± 0.05^{b} | 0.6 ± 0.06 | 0.5 ± 0.06^{b} | *-0.1 ± 0.06* |
| LDL-cholesterol (mmol/L) | 2.7 ± 0.2^{b} | 2.3 ± 0.1 | 2.1 ± 0.1^{b} | *-0.2 ± 0.1* | 2.4 ± 0.1 | 2.2 ± 0.1 | 2.1 ± 0.1^{b} | *-0.2 ± 0.1* |
| HDL-cholesterol (mmol/L) | 1.4 ± 0.05 | 1.5 ± 0.05^{b} | 1.4 ± 0.03 | *0.1 ± 0.05* | 1-5 ± 0.07^{b} | 1.3 ± 0.05 | 1.3 ± 0.05 | *0.0 ± 0.04* |
| HMW-adiponectin (mg/L)* | 11.6 ± 1.5^{c} | 7.0 ± 0.9 | 7.6 ± 0.8 | *0.1 ± 0.9* | 25.4 ± 3.7^{d} | 15.1 ± 3.0^{b} | 13.1 ± 2.4 | *3.2 ± 2.2* |
| C-Reactive Protein (mg/L)* | 2.4 ± 0.5^{b} | 1.1 ± 0.2 | 1.5 ± 0.5 | *0.2 ± 0.5* | 0.5 ± 0.1^{d} | 0.7 ± 0.2^{b} | 0.6 ± 0.1^{b} | *-0.9 ± 0.4* |
| Carotid IMT (mm) | .37 ± .01 | .37 ± .01 | .36 ± .01 | - *.01 ± .01* | .36 ± .01 ^{d} | .35 ± .01^{d} | .35 ± .01^{c} | - *.02 ± .01* |
| Systolic Blood Pressure (mmHg) | 112 ± 3 | 111 ± 2 | 112 ± 3 | *-3 ± 3* | 109 ± 1 | 111 ± 2 | 111 ± 2 | *-2 ± 2* |
| Diastolic Blood Pressure (mmHg) | 76 ± 3 | 72 ± 1 | 73 ± 2 | *1 ± 2* | 70 ± 1 | 72 ± 1 | 69 ± 2 | *-2 ± 2* |
| **DXA** BMD (g/cm²) | 1.18 ± 0.03 | 1.18 ± 0.03 | 1.19 ± 0.03^{c} | *0.02 ± 0.01* | 1.19 ± 0.03 | 1.18 ± 0.03 | 1.20 ± 0.03^{b} | *0.02 ± 0.01* |
| Lean Mass (Kg)^{#} | 34.8 ± 1.0 | 35.2 ± 0.9 | 35.3 ± 0.9 | *0.2 ± 0.3* | 35.3 ± 1.0 | 35.6 ± 1.2 | 35.4 ± 1.1 | *-0.2 ± 0.3* |
| Fat Mass (Kg)^{#} | 20.6 ± 1.6 | 20.9 ± 1.7 | 21.4 ± 1.8 | *0.8 ± 1.0* | 20.4 ± 1.8 | 19.8 ± 1.8 | 18.4 ± 2.1 | *-1.8 ± 1.2^{e}* |
| **Abd MRI** Subc Fat (cm²) | 138 ± 19 | 150 ± 22 | 152 ± 21 | *5 ± 15* | 142 ± 18 | 148 ± 21 | 138 ± 22 | *-12 ± 12* |
| Visceral Fat (cm²) | 41 ± 5 | 40 ± 5 | 37 ± 3 | *-6 ± 4* | 33 ± 2^{c} | 34 ± 2^{b} | 31 ± 2^{c} | *-18 ± 5^{g}* |
| Liver Fat (%) | 20 ± 2 | 17 ± 1 | 17 ± 2 | *0 ± 1* | 10 ± 1^{d} | 9 ± 1^{d} | 11 ± 1^{d} | *-6 ± 2^{f}* |

| | | | | | | | | |
|---|---|---|---|---|---|---|---|---|
| Values are mean ± SEM. SHBG, sex hormone-binding globulin; HOMA-IR: homeostasis model assessment insulin resistance; oGTT, oral glucose tolerance test; GGT, gamma-glutamyl transpeptidase; HMW, high molecular weight; IMT, intima-media thickness; DXA, dual X-ray absorptiometry; Abd MRI, abdominal magnetic resonance imaging; Subc, subcutaneous. ^ 100% of these girls had pseudo-menses within 28 days, following the intake of placebo pills for 7/28 days. *Indicative values in healthy adolescents (n=18; age 16.9 ± 0.3 yr; BMI 21.1 ± 0.6 Kg/m²); SHBG 59 ± 5 nmol/L; testosterone 0.7 ± 0.1 nmol/L; androstenedione 3.4 ± 0.2 nmol/L; fasting insulin 21.0 ± 2.1 pmol/L; HOMA-IR 0.7 ± 0.1; GGT 12 ±1 IU/L; HMW-adiponectin 12.7 ± 1.6 mg/L; CRP 0.7 ± 0.2 mg/L. "Indicative DXA values in healthy adolescents, matched for age and height (n=41): lean mass 35.1 ± 1.0 Kg; fat mass 17.6 ± 1.4 Kg Indicative MRI values in healthy adolescents (n=15; age 15.7 ± 0.3 yr; BMI 22.1 ± 0.8 Kg/m²): subc fat 97 ± 11 cm²; visceral fat 32 ± 2 cm²; hepatic fat 12 ± 1 %. ^{a}no significant differences between randomised subgroups at start. ^{b}p ≤0.05, ^{c}p≤0.01 and ^{d}p ≤0.001 for within-subgroup changes (Δ) from start. ^{e}p ≤0.05, | | | | | | | | |

## Claims

1. A pharmaceutical composition comprising spironolactone, pioglitazone and metformin for use in treating hepatic and/or visceral fat excess.

2. The pharmaceutical composition for use according to claim 1, in treating liver steatosis.

3. The pharmaceutical composition for use according to any one of of the preceding claims, in treating liver steatosis in adolescent girls or women of childbearing age having oligo-ovulation.

4. The pharmaceutical composition for use according to any one of the preceding claims, wherein the liver steatosis is not associated with being overweight or being obese.

5. The pharmaceutical composition for use according to any one of claims 3 or 4, wherein the adolescent girls or women of childbearing age exhibit androgen excess.

6. The pharmaceutical composition for use according to any one of claims 3 to 5, wherein the adolescent girls or women of childbearing age exhibit hyperinsulinemia, low adiponectin, high C reactive protein levels and/or high gonadotropin levels

7. The pharmaceutical composition for use according to any one of the preceding claims, wherein each of spironolactone, pioglitazone and metformin are administered sequentially in separate single delivery forms.

8. The pharmaceutical composition for use according to any one of the preceding claims, wherein two of spironolactone, pioglitazone and metformin are administered in a single delivery form and the remaining compound is administered in a separate delivery form.

9. The pharmaceutical composition for use according to claim 8, wherein the single delivery forms are administered at the same time or within 5 minutes to 1 hour of each other.

10. The pharmaceutical composition for use according to claim 9, wherein the single delivery forms are administered within 15 minutes to 30 minutes of each other.

11. The pharmaceutical composition for use according to any one of the preceding claims, wherein a daily dose of said pharmaceutical composition comprises between 25 and 100 mg spironolactone.

12. The pharmaceutical composition for use according to any one of the preceding claims, wherein a daily dose of said composition comprises between 5 and 15 mg pioglitazone.

13. The pharmaceutical composition for use according to any one of the preceding claims, wherein a daily dose of said composition comprises between 500 and 1500 mg metformin.

14. The pharmaceutical composition for use according to any one of the preceding claims, wherein a daily dose of said composition comprises between 25 and 100 mg spironolactone, between 5 and 15 mg pioglitazone and between 500 and 1500 mg metformin.

15. The pharmaceutical composition for use according to any one of the preceding claims, wherein a daily dose of said composition comprises 50 mg spironolactone, 7.5 mg pioglitazone and 850 mg metformin.
